# EUROPEAN PATENT APPLICATION

(11) **EP 4 349 825 A1**
(43) Date of publication of application: **10.04.2024**
(21) Application number: 22828823.9
(22) Date of filing: 24.06.2022
(51) Int. Cl.: C07D 401/12, A61K 31/4439, A61K 31/506, A61K 31/444, A61K 31/4436, A61P 35/00, A61P 37/00, C07D 401/14, C07D 417/14, C07D 405/14

(54) **NOVEL PYRIDINE DERIVATIVE COMPOUND AS RON INHIBITOR**

(30) Priority: 24.06.2021 KR 20210082699
(71) Applicant: LG Chem, Ltd., Yeongdeungpo-gu Seoul 07336 (KR)
(72) Inventor: HAM, Young Jin, Daejeon 34122 (KR); CHO, Joong Heui, Daejeon 34122 (KR); YOON, Hong Bin, Daejeon 34122 (KR); KIM, Jung Joon, Daejeon 34122 (KR); KWAK, Young Shin, Daejeon 34122 (KR); KIM, Gyeong Hwan, Daejeon 34122 (KR); LEE, Ju Hyun, Daejeon 34122 (KR)
(74) Representative: Hoffmann Eitle
(86) International application number: PCT/KR2022/009037
(87) International publication number: WO 2022/270976

(57) **Abstract**

The present invention relates to a novel compound as a protein kinase inhibitor, in particular, a RON inhibitor.

## Description

### TECHNICAL FIELD

### Cross-reference to Related Applications

This application claims the benefit of the priority of Korean Patent Application No. 10-2021-0082699, filed on June 24, 2021, the disclosure of which is incorporated herein in its entirety by reference.

### Technical Field

The present invention relates to a novel compound as a protein kinase inhibitor. Specifically, the present invention relates to a novel compound as a RON inhibitor.

### BACKGROUND ART

At least 400 protein kinases that catalyze a phosphoryl transfer reaction from adenosine triphosphate (ATP) to a protein substrate are known. In a target protein to which the phosphoryl group is transferred, a specific amino acid is tyrosine, serine, or threonine, so that protein kinases are commonly referred to as protein tyrosine kinases (PTKs) or serine/threonine kinases (STKs).

The protein kinases constitute a large family of structurally related enzymes that are responsible for the control of a wide variety of signaling pathways within the cell. The signaling pathways include many other signaling pathways by the transfer of the phosphoryl group to the target protein. These phosphorylation events act as molecular on/off switches that can regulate the biological function of the target protein or protein complex. The appropriate protein kinase functions in signaling pathways are to activate or inactivate metabolic enzymes, regulatory proteins, receptors, cytoskeletal proteins, ion channels and pumps, transcription factors, and the like. Uncontrolled signaling due to defective control of protein phosphorylation has been implicated in a number of diseases, including, inflammation, cancer, allergy/asthma, diseases of the immune system, diseases of the central nervous system, angiogenesis, and the like.

Almost all kinases contain a similar 250-300 amino acid catalytic domain. The kinases may be categorized by the substrates they phosphorylate, and sequence motifs have been identified that generally correspond to each of these kinase families.

Meanwhile, a receptor originated from nantes (RON), which is one of tyrosine protein kinase receptors, is also referred to as a macrophage stimulating 1 receptor (MST1R), and has been reported to promote the invasion and metastasis of cancer cells. Overexpression of RON is also known to appear in a variety of tumor types.

Activation of tyrosine protein kinases such as the RON in tumor cells increases the proliferation, invasion, and metastasis of tumor cells, and also increases the resistance of tumor cells to apoptosis and cytotoxic therapy. Therefore, a selective small molecule kinase modulator targeting a tyrosine protein kinase such as the RON is expected to have therapeutic potential for the treatment of cancers in which activation of RON receptors and the like plays a critical role in the development and progression of primary tumors and secondary metastases. Accordingly, continuous research is being conducted on various inhibitors for selectively inhibiting kinase activity in RON, one of the tyrosine proteins.

### DISCLOSURE OF THE INVENTION

### TECHNICAL PROBLEM

An aspect of the present invention provides a novel compound having protein kinase inhibitory activity.

Another aspect of the present invention provides a compound useful for the prevention or treatment of cancer.

Yet another aspect of the present invention provides a compound useful for the prevention or treatment of immune related diseases.

Still yet another aspect of the present invention provides a compound useful as a RON inhibitor.

### TECHNICAL SOLUTION

In order to achieve the above objective, according to an aspect of the present invention, there is provided a pyridine derivative compound of Formula 1 below or a pharmaceutically acceptable salt thereof.

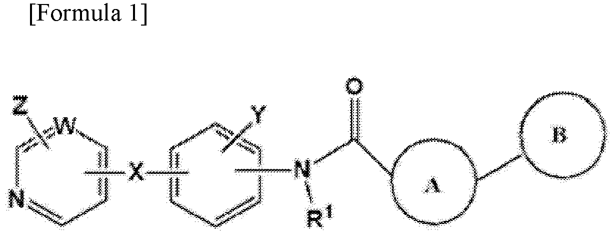

In Formula 1 above,
W above is N or CH,
X above is O,
Y above is selected from among hydrogen, halogen, C₁-C₆ alkyl, and C₃-C₆ cycloalkyl,
Z above is at least one selected from among hydrogen, halogen, C₁-C₆ alkyl, C₃-C₁₀ cycloalkyl, C₆-C₁₀ aryl, halogen-substituted C₁-C₆ alkyl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR²R³,
R¹ above is hydrogen or C₁-C₆ alkyl,
A above represents substituted or unsubstituted 5-membered heteroaryl containing one to four heterocyclic atoms selected from the group consisting of nitrogen, sulfur, and oxygen, wherein the substituent of the substituted 5-membered heteroaryl is halogen, amine, C₁-C₆ alkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol or NR²R³,
B above is C₃-C₁₀ heteroaryl or substituted C₃-C₁₀ heteroaryl, wherein the substituent is halogen, C₁-C₆ alkyl, halogen-substituted C₁-C₆ alkyl, C₁-C₆ alkoxy, hydroxy, or C₆-C₁₀ aryl,
R² and R³ above are each independently hydrogen, C₁-C₆ alkyl, C₃-C₆ cycloalkyl, substituted C₃-C₆ cycloalkyl, and
the halogens are each independently selected from the group consisting of F, C1, Br, and I.

According to another aspect of the present invention, there is provided a pharmaceutical composition including a compound of Formula 1 above or a pharmaceutically acceptable salt thereof, and a pharmaceutically acceptable carrier.

### ADVANTAGEOUS EFFECTS

The present invention can provide a novel compound or a pharmaceutically acceptable salt thereof which can be effectively used in the treatment of various immune related diseases, as anticancer drugs by inhibiting the activity of protein kinases, particularly, RON receptors, but the effects of the present invention are not limited thereto.

### MODE FOR CARRYING OUT THE INVENTION

According to an aspect, the present invention provides a novel pyridine derivative compound of Formula 1 as defined above or a pharmaceutically acceptable salt thereof.

In the present invention, the compound is intended to include a compound of Formula 1, a stereoisomer such as an enantiomer thereof and a diastereomer, a solvate, a prodrug, *etc.,* unless otherwise stated.

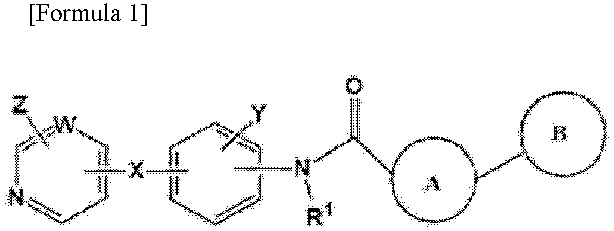

In Formula 1 above,
W above is N or CH,
X above is O,
Y above is selected from among hydrogen, halogen, C₁-C₆ alkyl, and C₃-C₆ cycloalkyl,
Z above is at least one selected from among hydrogen, halogen, C₁-C₆ alkyl, C₃-C₁₀ cycloalkyl, C₆-C₁₀ aryl, halogen-substituted C₁-C₆ alkyl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR²R³,
R¹ above is hydrogen or C₁-C₆ alkyl,
A above represents substituted or unsubstituted 5-membered heteroaryl containing one to four heterocyclic atoms selected from the group consisting of nitrogen, sulfur, and oxygen, wherein the substituent of the substituted 5-membered heteroaryl is halogen, amine, C₁-C₆ alkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol or NR²R³,
B above is C₃-C₁₀ heteroaryl or substituted C₃-C₁₀ heteroaryl, wherein the substituent is halogen, C₁-C₆ alkyl, halogen-substituted C₁-C₆ alkyl, C₁-C₆ alkoxy, hydroxy, or C₆-C₁₀ aryl, and
R² and R³ above are each independently hydrogen, C₁-C₆ alkyl, C₃-C₆ cycloalkyl, substituted C₃-C₆ cycloalkyl,
wherein the halogens are each independently selected from the group consisting of F, Cl, Br, and I.

In the present specification, the term "substitution" means that a hydrogen atom bonded to a carbon atom in a structure is replaced with another substituent, and the substituted position is not limited as long as it is a position at which the hydrogen atom is substituted, that is, a position at which a substituent can be substituted, and when the hydrogen atoms are substituted at two or more positions, two or more substituents may be the same as or different from each other.

In the present specification, unless otherwise defined, the "substituent" may be one or more selected from the group consisting of deuterium, halogen, hydroxy, C₁-C₁₀ alkyl, C₃-C₁₂ cycloalkyl, C₁-C₁₀ alkoxy, C₅-C₁₂ aryloxy, C₁-C₁₀ alkylthioxy, C₅-C₁₂ arylthioxy, C₁-C₁₀ alkylsulfoxy, C₅-C₁₂ arylsulfoxy, C₁-C₁₀ haloalkyl, C₂-C₂₀ alkenyl, C₀-C₁₀ amine, nitrile, nitro, imide, amide, oxo, carbonyl, carboxylic acid, carbamoyl, ester, C₅-C₁₂ aryl, and C₅-C₁₂ heteroaryl.

In the present specification, the "alkyl" may be linear or branched, and preferably has 1 to 20 carbon atoms unless otherwise defined. Examples of the alkyl include methyl, ethyl, propyl, n-propyl, isopropyl, butyl, n-butyl, isobutyl, tert-butyl, sec-butyl, 1-methylbutyl, ethylbutyl, pentyl, n-pentyl, isopentyl, neopentyl, tert-pentyl, hexyl, n-hexyl, methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, dimethylpropyl, isohexyl, 2-methylpentyl, 4-methylhexyl, 5-methylhexyl, and the like, but are not limited thereto.

In the present specification, the "cycloalkyl" may mean a cyclic saturated hydrocarbon, and preferably has 3 to 20 carbon atoms unless otherwise defined. Examples of the cycloalkyl include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, the "alkoxy" group may be linear, branched, or cyclic, and preferably has 1 to 20 carbon atoms unless otherwise defined. Examples of the alkoxy group include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the "alkenyl" group may be linear or branched, and preferably has 2 to 20 carbon atoms unless otherwise defined. Examples of the alkenyl group include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stylbenyl group, a styrenyl group, and the like, but are not limited thereto.

In the present specification, the "aryl" may be monoaryl, biaryl, or polycyclic aryl having three or more rings, and when the aryl includes two or more cyclic structures, each ring may be fused or included in a spiro form, and the aryl preferably has 5 to 12 carbon atoms unless otherwise defined. Examples of the aryl group include a phenyl group, a biphenyl group, a terphenyl group, a naphthyl group, an anthracenyl group, a phenanthryl group, a pyrenyl group, a perylenyl group, and the like, but are not limited thereto.

In the present specification, the "heterocyclic atom" means a non-carbon atom contained in the ring. Unless otherwise defined, the heterocyclic atom may include one or more atoms selected from among oxygen, nitrogen, selenium and sulfur.

In the present specification, the "heterocycloalkyl" may mean a cyclic saturated hydrocarbon containing a heterocyclic atom, and preferably have 2 to 20 carbon atoms unless otherwise defined.

In the present specification, the "heteroaryl" may mean an aromatic hydrocarbon containing a heterocyclic atom. The heteroaryl may be monocyclic or polycyclic, and when the heteroaryl includes two or more cyclic structures, each ring may be fused or included in a spiro form, and the heteroaryl preferably has 5 to 12 carbon atoms unless otherwise defined. Examples of the heteroaryl include a thiophene group, a furanyl group, a pyrrole group, an imidazolyl group, a thiazolyl group, an oxazolyl group, an oxadiazolyl group, a pyridyl group, a bipyridyl group, a pyrimidyl group, a triazinyl group, a triazolyl group, an acridyl group, a pyridazinyl group, a pyrazinyl group, a quinolinyl group, a quinazolinyl group, a quinoxalinyl group, a phthalazinyl group, a pyridopyrimidyl group, a pyridopyrazinyl group, a pyrazinopyrazinyl group, an isoquinolinyl group, an indolyl group, a carbazolyl group, a benzoxazolyl group, a benzimidazolyl group, a benzothiazolyl group, and the like, but are not limited thereto.

According to an embodiment, the present invention provides a novel pyridine derivative compound of Formula 2 below or a pharmaceutically acceptable salt thereof.

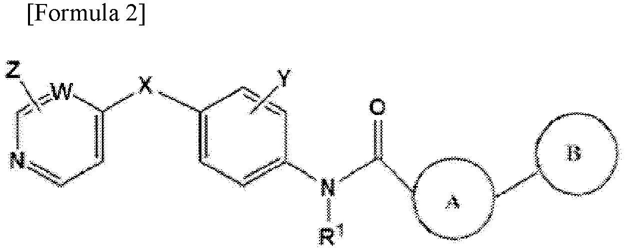

W above is N or CH, Z is at least one selected from hydrogen, halogen, C₁-C₆ alkyl, and NR²R³, wherein R² and R³ above are each independently hydrogen, C₁-C₆ alkyl, C₃-C₆ cycloalkyl, substituted C₃-C₆ cycloalkyl, and the remaining substituents are the same as defined in Formula 1.

According to an embodiment, A above may include a heteroaryl group selected from the group consisting of pyrazole, imidazole, thiazole, and thiophene, wherein the heteroaryl group may be substituted or unsubstituted, and the substituent of the substituted heteroaryl group may be halogen, amine, C₁-C₆ alkyl, halogen-substituted C₁-C₆ alkyl, or C₆-C₁₀ aryl.

According to an embodiment, in Formula 1 above, B above may include pyridine, C₁-C₃ alkyl-substituted pyridine, halogen-substituted pyridine, halogenated C₁-C₃ alkyl-substituted pyridine, C₁-C₃ alkoxy-substituted pyridine, hydroxy pyridine, pyrimidine, C₁-C₃ alkyl-substituted pyrimidine, halogen-substituted pyrimidine, hydroxy pyrimidine, pyridazine, alkyl-substituted pyridazine, halogen-substituted pyridazine, pyrazole, thiazole, alkyl-substituted thiazole, or C₆-C₁₀ aryl-substituted thiazole.

According to an embodiment, in Formula 1 above, Y above is fluoro.

According to an embodiment, in Formula 1 above, Z above is present at two or more positions.

According to an embodiment, in Formula 1 above, Z above is present at two positions.

According to an embodiment, in Formula 1 above, R¹ above is hydrogen.

According to an embodiment, the compound of Formula 1 above may be selected from the group consisting of N-(4-((2-amino-3-chloropyridin-4-yl)oxy)-3-fluorophenyl)-5-ethyl-1-(pyrimidin-5-yl)-1H-pyrazole-4-carboxamide, N-(4-((2-amino-3-chloropyridin-4-yl)oxy)-3-fluorophenyl)-1-(pyridin-2-yl)-5-(trifluoromethyl)-1H-pyrazole-4-carboxamide, N-(4-((2-amino-3-chloropyridin-4-yl)oxy)-3-fluorophenyl)-1-(4-phenylthiazol-2-yl)-5-(trifluoromethyl)-1H-pyrazole-4-carboxamide, N-(4-((2-amino-3-chloropyridin-4-yl)oxy)-3-fluorophenyl)-5-ethyl-1-(pyridin-4-yl)-1H-pyrazole-4-carboxamide, N-(4-((2-amino-3-chloropyridin-4-yl)oxy)-3-fluorophenyl)-5-ethyl-1-(pyridin-3-yl)-1H-pyrazole-4-carboxamide, N-(4-((2-amino-3-chloropyridin-4-yl)oxy)-3-fluorophenyl)-1-(pyrimidin-5-yl)-5-(trifluoromethyl)-1H-pyrazole-4-carboxamide, N-(4-((2-amino-3-chloropyridin-4-yl)oxy)-3-fluorophenyl)-5-methyl-1-(pyrimidin-5-yl)-1H-pyrazole-4-carboxamide, N-(4-((2-amino-3-chloropyridin-4-yl)oxy)-3-fluorophenyl)-1-(3-fluoropyridin-2-yl)-5-(trifluoromethyl)-1H-pyrazole-4-carboxamide, N-(4-((2-amino-3-chloropyridin-4-yl)oxy)-3-fluorophenyl)-1-(3-methylpyridin-2-yl)-5-(trifluoromethyl)-1H-pyrazole-4-carboxamide, N-(4-((2-amino-3-chloropyridin-4-yl)oxy)-3-fluorophenyl)-1-(3-chloropyridin-2-yl)-5-(trifluoromethyl)-1H-pyrazole-4-carboxamide, N-(4-((2-amino-3-chloropyridin-4-yl)oxy)-3-fluorophenyl)-5-(trifluoromethyl)-1-(3-(trifluoromethyl)pyridin-2-yl)-1H-pyrazole-4-carboxamide, N-(4-((2-amino-3-chloropyridin-4-yl)oxy)-3-fluorophenyl)-1-(4-methylpyridin-2-yl)-5-(trifluoromethyl)-1H-pyrazole-4-carboxamide, N-(4-((2-amino-3-chloropyridin-4-yl)oxy)-3-fluorophenyl)-1-(5-fluoropyridin-2-yl)-5-(trifluoromethyl)-1H-pyrazole-4-carboxamide, N-(4-((2-amino-3-chloropyridin-4-yl)oxy)-3-fluorophenyl)-1-(4-chloropyridin-2-yl)-5-(trifluoromethyl)-1H-pyrazole-4-carboxamide, N-(4-((2-amino-3-chloropyridin-4-yl)oxy)-3-fluorophenyl)-1-(3-methoxypyridin-2-yl)-5-(trifluoromethyl)-1H-pyrazole-4-carboxamide, N-(4-((2-amino-3-chloropyridin-4-yl)oxy)-3-fluorophenyl)-1-(5-methylpyridin-2-yl)-5-(trifluoromethyl)-1H-pyrazole-4-carboxamide, N-(4-((2-amino-3-chloropyridin-4-yl)oxy)-3-fluorophenyl)-1-(6-methylpyridin-2-yl)-5-(trifluoromethyl)-1H-pyrazole-4-carboxamide, N-(4-((2-amino-3-chloropyridin-4-yl)oxy)-3-fluorophenyl)-1-(pyridin-3-yl)-5-(trifluoromethyl)-1H-pyrazole-4-carboxamide, N-(4-((2-amino-3-chloropyridin-4-yl)oxy)-3-fluorophenyl)-1-(pyridin-4-yl)-5-(trifluoromethyl)-1H-pyrazole-4-carboxamide, N-(4-((2-amino-3-chloropyridin-4-yl)oxy)-3-fluorophenyl)-1-(pyridazin-3-yl)-5-(trifluoromethyl)-1H-pyrazole-4-carboxamide, N-(4-((2-amino-3-chloropyridin-4-yl)oxy)-3-fluorophenyl)-1-(pyrimidin-2-yl)-5-(trifluoromethyl)-1H-pyrazole-4-carboxamide, N-(4-((2-amino-3-chloropyridin-4-yl)oxy)-3-fluorophenyl)-1-(pyrimidin-4-yl)-5-(trifluoromethyl)-1H-pyrazole-4-carboxamide, N-(4-((2-amino-3-chloropyridin-4-yl)oxy)-3-fluorophenyl)-1-(4-methylpyrimidin-2-yl)-5-(trifluoromethyl)-1H-pyrazole-4-carboxamide, N-(4-((2-amino-3-chloropyridin-4-yl)oxy)-3-fluorophenyl)-1-(3-ethoxypyridin-2-yl)-5-(trifluoromethyl)-1H-pyrazole-4-carboxamide, N-(4-((2-amino-3-chloropyridin-4-yl)oxy)-3-fluorophenyl)-1-(4-fluoropyridin-2-yl)-5-(trifluoromethyl)-1H-pyrazole-4-carboxamide, N-(4-((2-amino-3-chloropyridin-4-yl)oxy)-3-fluorophenyl)-1-(5-fluoropyrimidin-2-yl)-5-(trifluoromethyl)-1H-pyrazole-4-carboxamide, N-(4-((2-amino-3-chloropyridin-4-yl)oxy)-3-fluorophenyl)-1-(5-hydroxypyrimidin-2-yl)-5-(trifluoromethyl)-1H-pyrazole-4-carboxamide, N-(4-((2-amino-3-iodopyridin-4-yl)oxy)-3-fluorophenyl)-1-(3-fluoropyridin-2-yl)-5-(trifluoromethyl)-1H-pyrazole-4-carboxamide, N-(4-((2-amino-3-chloropyridin-4-yl)oxy)-3-fluorophenyl)-1-(3,5-difluoropyridin-2-yl)-5-(trifluoromethyl)-1H-pyrazole-4-carboxamide, N-(4-((2-amino-3-chloropyridin-4-yl)oxy)-3-fluorophenyl)-1-(6-fluoropyridazin-3-yl)-5-(trifluoromethyl)-1H-pyrazole-4-carboxamide, N-(4-((2-aminopyridin-4-yl)oxy)-3-fluorophenyl)-1-(3-fluoropyridin-2-yl)-5-(trifluoromethyl)-1H-pyrazole-4-carboxamide, N-(4-((2-amino-3-chloropyridin-4-yl)oxy)-2-fluorophenyl)-1-(3-fluoropyridin-2-yl)-5-(trifluoromethyl)-1H-pyrazole-4-carboxamide, N-(4-((2-amino-3-(cyclohexylamino)pyridin-4-yl)oxy)-3-fluorophenyl)-1-(3-fluoropyridin-2-yl)-5-(trifluoromethyl)-1H-pyrazole-4-carboxamide, N-(4-((2-amino-3-(isopropylamino)pyridin-4-yl)oxy)-3-fluorophenyl)-1-(3-fluoropyridin-2-yl)-5-(trifluoromethyl)-1H-pyrazole-4-carboxamide, N-(4-((2-amino-3-chloropyridin-4-yl)oxy)-3-fluorophenyl)-5-ethyl-1-(3-fluoropyridin-2-yl)-1H-pyrazole-4-carboxamide, N-(4-((2-amino-3-chloropyridin-4-yl)oxy)-3-fluorophenyl)-1-(3-chloropyridin-2-yl)-5-ethyl-1H-pyrazole-4-carboxamide, N-(4-((2-amino-3-iodopyridin-4-yl)oxy)-3-fluorophenyl)-5-ethyl-1-(3-fluoropyridin-2-yl)-1H-pyrazole-4-carboxamide, N-(4-((2-amino-3-iodopyridin-4-yl)oxy)-3-fluorophenyl)-1-(3-chloropyridin-2-yl)-5-ethyl-1H-pyrazole-4-carboxamide, N-(4-((2-amino-3-chloropyridin-4-yl)oxy)-3 -fluorophenyl)-1'-methyl-5-(trifluoromethyl)-1'H-[1,4'-bipyrazole]-4-carboxamide, N-(4-((2-amino-3-chloropyridin-4-yl)oxy)-3-fluorophenyl)-1-(4-methylthiazol-2-yl)-5-(trifluoromethyl)-1H-pyrazole-4-carboxamide, N-[4-(6-amino-5-chloro-pyrimidin-4-yl)oxy-3-fluoro-phenyl]-4-phenyl-thiazole-2-carboxamide, N-[4-(6-amino-5-chloro-pyrimidin-4-yl)oxy-3-fluoro-phenyl]-1-(1-methylpyrazol-4-yl)-5-(trifluoromethyl)pyrazole-4-carboxamide, N-(4-((6-amino-5-chloropyrimidin-4-yl)oxy)-3-fluorophenyl)-1-(pyridin-2-yl)-5-(trifluoromethyl)-1H-pyrazole-4-carboxamide, N-[4-(6-amino-5-chloro-pyrimidin-4-yl)oxy-3-fluoro-phenyl]-1-pyrimidin-5-yl-5-(trifluoromethyl)pyrazole-4-carboxamide, N-[4-(6-amino-5-chloro-pyrimidin-4-yl)oxy-3-fluoro-phenyl]-1-(3-fluoro-2-pyridyl)-5-(trifluoromethyl)pyrazole-4-carboxamide, N-[4-(6-amino-5-chloro-pyrimidin-4-yl)oxy-3-fluoro-phenyl]-1-(3-methyl-2-pyridyl)-5-(trifluoromethyl)pyrazole-4-carboxamide, N-[4-(6-amino-5-chloro-pyrimidin-4-yl)oxy-3-fluoro-phenyl]-1-(4-methyl-2-pyridyl)-5-(trifluoromethyl)pyrazole-4-carboxamide, N-[4-(6-amino-5-chloro-pyrimidin-4-yl)oxy-3-fluoro-phenyl]-1-(5-methyl-2-pyridyl)-5-(trifluoromethyl)pyrazole-4-carboxamide, N-[4-(6-amino-5-chloro-pyrimidin-4-yl)oxy-3-fluoro-phenyl]-1-(6-methyl-2-pyridyl)-5-(trifluoromethyl)pyrazole-4-carboxamide, N-[4-(6-amino-5-chloro-pyrimidin-4-yl)oxy-3-fluoro-phenyl]-1-(5-fluoro-2-pyridyl)-5-(trifluoromethyl)pyrazole-4-carboxamide, N-[4-(6-amino-5-chloro-pyrimidin-4-yl)oxy-3-fluoro-phenyl]-1-(3-chloro-2-pyridyl)-5-(trifluoromethyl)pyrazole-4-carboxamide, N-[4-(6-amino-5-chloro-pyrimidin-4-yl)oxy-3-fluoro-phenyl]-1-(4-chloro-2-pyridyl)-5-(trifluoromethyl)pyrazole-4-carboxamide, N-[4-(6-amino-5-chloro-pyrimidin-4-yl)oxy-3-fluoro-phenyl]-5-(trifluoromethyl)-1-[3-(trifluoromethyl)-2-pyridyl]pyrazole-4-carboxamide, N-[4-(6-amino-5-chloro-pyrimidin-4-yl)oxy-3-fluoro-phenyl]-1-(3-methoxy-2- pyridyl)-5-(trifluoromethyl)pyrazole-4-carboxamide, N-[4-(6-amino-5-chloro-pyrimidin-4-yl)oxy-3-fluoro-phenyl]-1-(4-ethyl-2-pyridyl)-5-(trifluoromethyl)pyrazole-4-carboxamide, N-[4-(6-amino-5-chloro-pyrimidin-4-yl)oxy-3-fluoro-phenyl]-1-(3-pyridyl)-5-(trifluoromethyl)pyrazole-4-carboxamide, N-[4-(6-amino-5-chloro-pyrimidin-4-yl)oxy-3-fluoro-phenyl]-1-(4-pyridyl)-5-(trifluoromethyl)pyrazole-4-carboxamide, N-[4-(6-amino-5-chloro-pyrimidin-4-yl)oxy-3-fluoro-phenyl]-1-(3-ethyl-2-pyridyl)-5-(trifluoromethyl)pyrazole-4-carboxamide, N-[4-(6-amino-5-chloro-pyrimidin-4-yl)oxy-3-fluoro-phenyl]-1-pyrimidin-2-yl-5-(trifluoromethyl)pyrazole-4-carboxamide, N-[4-(6-amino-5-chloro-pyrimidin-4-yl)oxy-3-fluoro-phenyl]-1-pyridazin-3-yl-5-(trifluoromethyl)pyrazole-4-carboxamide, N-[4-(6-amino-5-chloro-pyrimidin-4-yl)oxy-3-fluoro-phenyl]-1-pyrimidin-4-yl-5-(trifluoromethyl)pyrazole-4-carboxamide, N-[4-(6-amino-5-chloro-pyrimidin-4-yl)oxy-3-fluoro-phenyl]-1-(4-methylpyrimidin-2-yl)-5-(trifluoromethyl)pyrazole-4-carboxamide, N-[4-(6-amino-5-chloro-pyrimidin-4-yl)oxy-3-fluoro-phenyl]-1-(4-fluoro-2-pyridyl)-5-(trifluoromethyl)pyrazole-4-carboxamide, N-[4-(6-amino-5-chloro-pyrimidin-4-yl)oxy-3-fluoro-phenyl]-1-(4-hydroxy-2-pyridyl)-5-(trifluoromethyl)pyrazole-4-carboxamide, N-[4-[5-chloro-6-(cyclopropylamino)pyrimidin-4-yl]oxy-3-fluoro-phenyl]-1-(3-fluoro-2-pyridyl)-5-(trifluoromethyl)pyrazole-4-carboxamide, and N-[4-[5-chloro-6-(cyclopropylamino)pyrimidin-4-yl]oxy-3-fluorophenyl]-1-(3-chloro-2-pyridyl)-5-(trifluoromethyl)pyrazole-4-carboxamide, and the structures thereof are shown in Table 1 below.

**[Table 1]**

| | **Structure** | **IUPAC Name** |
|---|---|---|
| **Example1** | 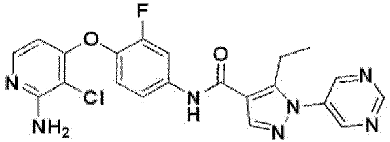 | N-(4-((2-amino-3-chloropyridin-4-yl)oxy)-3-fluorophenyl)-5-ethyl-1-(pyrimidin-5-yl)-1H-pyrazole-4-carboxamide |
| **Example2** | 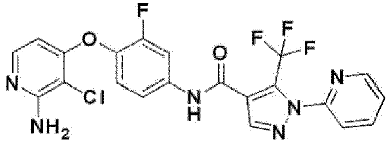 | N-(4-((2-amino-3-chloropyridin-4-yl)oxy)-3-fluorophenyl)-1-(pyridin-2-yl)-5-(trifluoromethyl)-1H-pyrazole-4-carboxamide |
| **Example3** | 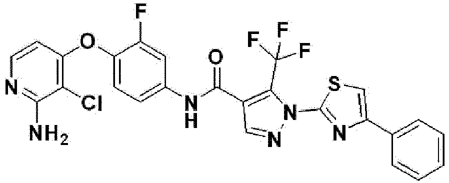 | N-(4-((2-amino-3-chloropyridin-4-yl)oxy)-3-fluorophenyl)-1-(4-phenylthiazol-2-yl)-5-(trifluoromethyl)-1H-pyrazole-4-carboxamide |
| **Example4** | 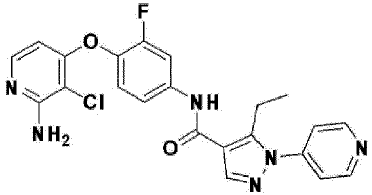 | N-(4-((2-amino-3-chloropyridin-4-yl)oxy)-3-fluorophenyl)-5-ethyl-1-(pyridin-4-yl)-1H-pyrazole-4-carboxamide |
| **Example5** | 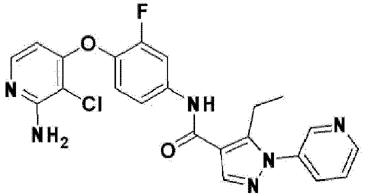 | N-(4-((2-amino-3-chloropyridin-4-yl)oxy)-3-fluorophenyl)-5-ethyl-1-(pyridin-3-yl)-1H-pyrazole-4-carboxamide |
| **Example6** | 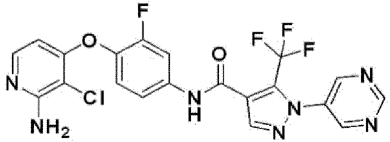 | N-(4-((2-amino-3-chloropyridin-4-yl)oxy)-3-fluorophenyl)-1-(pyrimidin-5-yl)-5-(trifluoromethyl)-1H-pyrazole-4-carboxamide |
| **Example7** | 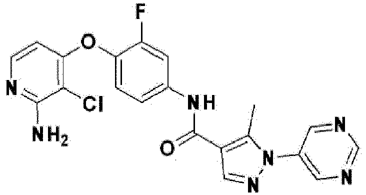 | N-(4-((2-amino-3-chloropyridin-4-yl)oxy)-3-fluorophenyl)-5-methyl-1-(pyrimidin-5-yl)-1H-pyrazole-4-carboxamide |
| **Example8** | 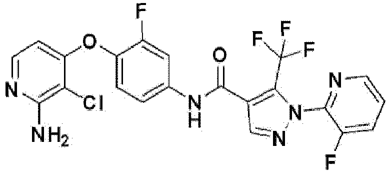 | N-(4-((2-amino-3-chloropyridin-4-yl)oxy)-3-fluorophenyl)-1-(3-fluoropyridin-2-yl)-5-(trifluoromethyl)-1H-pyrazole-4-carboxamide |
| **Example9** | 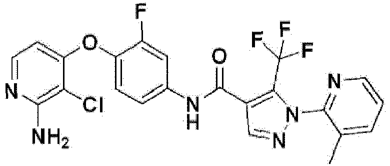 | N-(4-((2-amino-3-chloropyridin-4-yl)oxy)-3-fluorophenyl)-1-(3-methylpyridin-2-yl)-5-(trifluoromethyl)-1H-pyrazole-4-carboxamide |
| **Example10** | 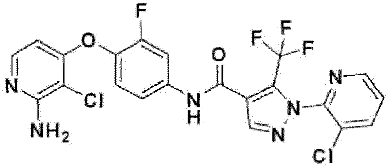 | N-(4-((2-amino-3-chloropyridin-4-yl)oxy)-3-fluorophenyl)-1-(3-chloropyridin-2-yl)-5- (trifluoromethyl)-1H-pyrazole-4-carboxamide |
| **Example11** | 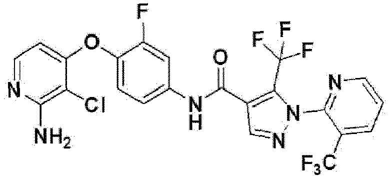 | N-(4-((2-amino-3-chloropyridin-4-yl)oxy)-3-fluorophenyl)-5-(trifluoromethyl)-1-(3-(trifluoromethyl)pyridin-2-yl)-1H-pyrazole-4-carboxamide |
| **Example12** | 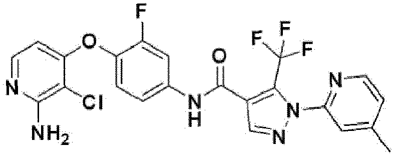 | N-(4-((2-amino-3-chloropyridin-4-yl)oxy)-3-fluorophenyl)-1-(4-methylpyridin-2-yl)-5-(trifluoromethyl)-1H-pyrazole-4-carboxamide |
| **Example13** | 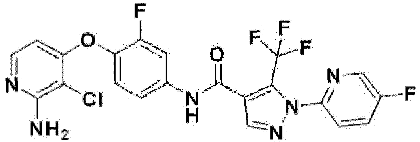 | N-(4-((2-amino-3-chloropyridin-4-yl)oxy)-3-fluorophenyl)-1-(5-fluoropyridin-2-yl)-5-(trifluoromethyl)-1H-pyrazole-4-carboxamide |
| **Example14** | 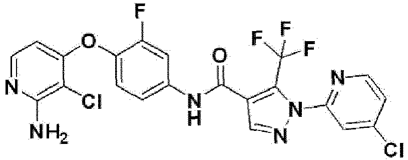 | N-(4-((2-amino-3-chloropyridin-4-yl)oxy)-3-fluorophenyl)-1-(4-chloropyridin-2-yl)-5-(trifluoromethyl)-1H-pyrazole-4-carboxamide |
| **Example15** | 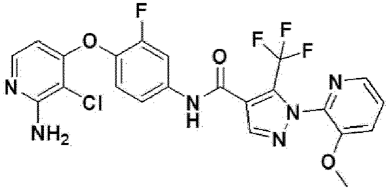 | N-(4-((2-amino-3-chloropyridin-4-yl)oxy)-3-fluorophenyl)-1-(3-methoxypyridin-2-yl)-5-(trifluoromethyl)-1H-pyrazole-4-carboxamide |
| **Example16** | 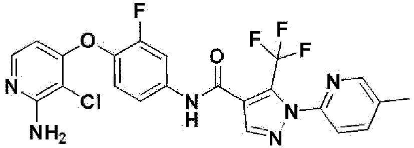 | N-(4-((2-amino-3-chloropyridin-4-yl)oxy)-3-fluorophenyl)-1-(5-methylpyridin-2-yl)-5-(trifluoromethyl)-1H-pyrazole-4-carboxamide |
| **Example17** | 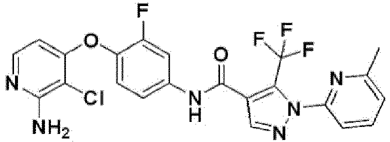 | N-(4-((2-amino-3-chloropyridin-4-yl)oxy)-3-fluorophenyl)-1-(6-methylpyridin-2-yl)-5-(trifluoromethyl)-1H-pyrazole-4-carboxamide |
| **Example18** | 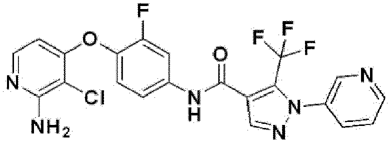 | N-(4-((2-amino-3-chloropyridin-4-yl)oxy)-3-fluorophenyl)-1-(pyridin-3-yl)-5-(trifluoromethyl)-1H-pyrazole-4-carboxamide |
| **Example19** | 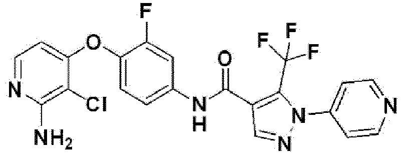 | N-(4-((2-amino-3-chloropyridin-4-yl)oxy)-3-fluorophenyl)-1-(pyridin-4-yl)-5-(trifluoromethyl)-1H-pyrazole-4-carboxamide |
| **Example20** | 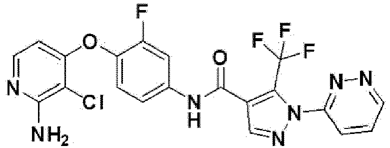 | N-(4-((2-amino-3-chloropyridin-4-yl)oxy)-3-fluorophenyl)-1-(pyridazin-3-yl)-5-(trifluoromethyl)-1H-pyrazole-4-carboxamide |
| **Example21** | 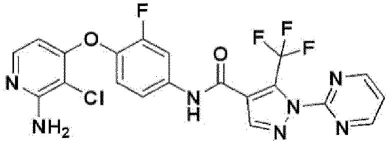 | N-(4-((2-amino-3-chloropyridin-4-yl)oxy)-3-fluorophenyl)-1-(pyrimidin-2-yl)-5-(trifluoromethyl)-1H-pyrazole-4-carboxamide |
| **Example22** | 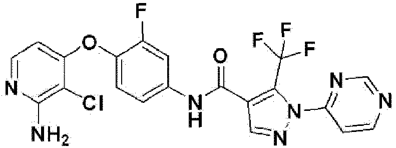 | N-(4-((2-amino-3-chloropyridin-4-yl)oxy)-3-fluorophenyl)-1-(pyrimidin-4-yl)-5-(trifluoromethyl)-1H-pyrazole-4-carboxamide |
| **Example23** | 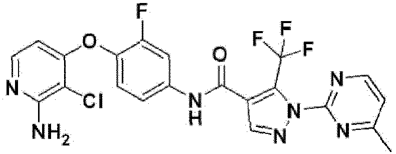 | N-(4-((2-amino-3-chloropyridin-4-yl)oxy)-3-fluorophenyl)-1-(4-methylpyrimidin-2-yl)-5-(trifluoromethyl)-1H-pyrazole-4-carboxamide |
| **Example24** | 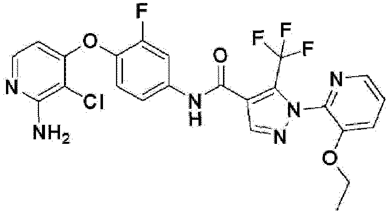 | N-(4-((2-amino-3-chloropyridin-4-yl)oxy)-3-fluorophenyl)-1-(3-ethoxypyridin-2-yl)-5- (trifluoromethyl)-1H-pyrazole-4-carboxamide |
| **Example25** | 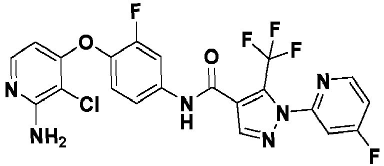 | N-(4-((2-amino-3-chloropyridin-4-yl)oxy)-3-fluorophenyl)-1-(4-fluoropyridin-2-yl)-5-(trifluoromethyl)-1H-pyrazole-4-carboxamide |
| **Example26** | 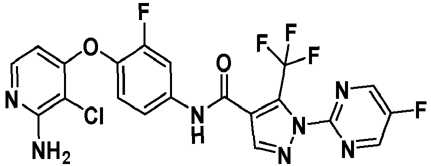 | N-(4-((2-amino-3-chloropyridin-4-yl)oxy)-3-fluorophenyl)-1-(5-fluoropyrimidin-2-yl)-5- (trifluoromethyl)-1H-pyrazole-4-carboxamide |
| **Example27** | 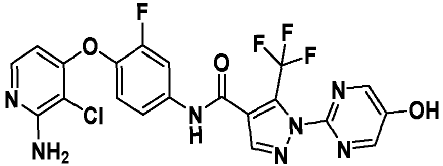 | N-(4-((2-amino-3-chloropyridin-4-yl)oxy)-3-fluorophenyl)-1-(5-hydroxypyrimidin-2-yl)-5-(trifluoromethyl)-1H-pyrazole-4-carboxamide |
| **Example28** | 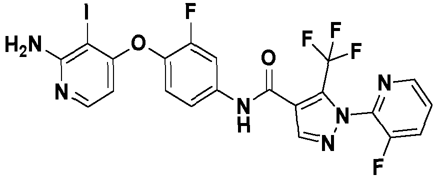 | N-(4-((2-amino-3-iodopyridin-4-yl)oxy)-3-fluorophenyl)-1-(3-fluoropyridin-2-yl)-5-(trifluoromethyl)-1H-pyrazole-4-carboxamide |
| **Example29** | 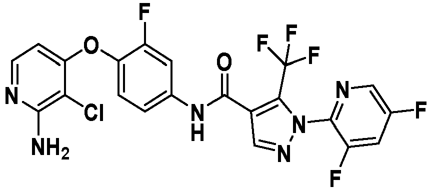 | N-(4-((2-amino-3-chloropyridin-4-yl)oxy)-3-fluorophenyl)-1-(3,5-difluoropyridin-2-yl)-5- (trifluoromethyl)-1H-pyrazole-4-carboxamide |
| **Example30** | 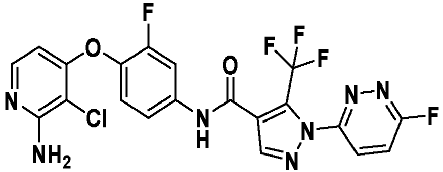 | N-(4-((2-amino-3-chloropyridin-4-yl)oxy)-3-fluorophenyl)-1-(6-fluoropyridazin-3-yl)-5-(trifluoromethyl)-1H-pyrazole-4-carboxamide |
| **Example31** | 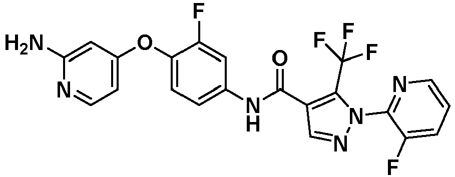 | N-(4-((2-aminopyridin-4-yl)oxy)-3-fluorophenyl)-1-(3-fluoropyridin-2-yl)-5-(trifluoromethyl)-1H-pyrazole-4-carboxamide |
| **Example32** | 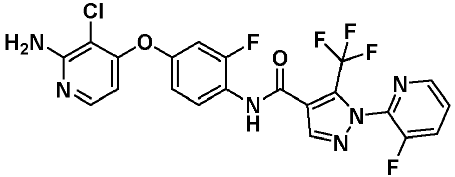 | N-(4-((2-amino-3-chloropyridin-4-yl)oxy)-2-fluorophenyl)-1-(3-fluoropyridin-2-yl)-5-(trifluoromethyl)-1H-pyrazole-4-carboxamide |
| **Example33** | 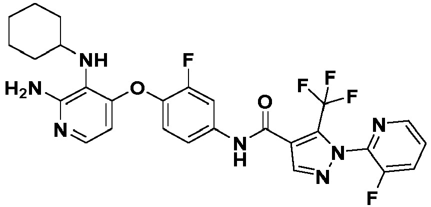 | N-(4-((2-amino-3-(cyclohexylamino)pyridin-4-yl)oxy)-3-fluorophenyl)-1-(3-fluoropyridin-2-yl)-5-(trifluoromethyl)-1H-pyrazole-4-carboxamide |
| **Example34** | 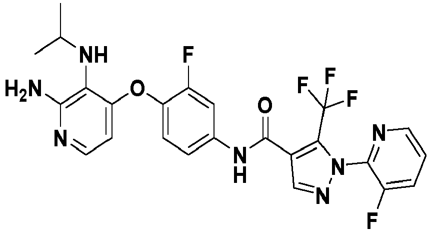 | N-(4-((2-amino-3-(isopropylamino)pyridin-4-yl)oxy)-3-fluorophenyl)-1-(3-fluoropyridin-2-yl)-5-(trifluoromethyl)-1H-pyrazole-4-carboxamide |
| **Example35** | 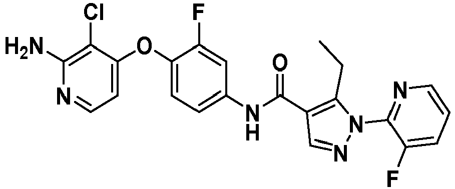 | N-(4-((2-amino-3-chloropyridin-4-yl)oxy)-3-fluorophenyl)-5-ethyl-1-(3-fluoropyridin-2-yl)-1H-pyrazole-4-carboxamide |
| **Example36** | 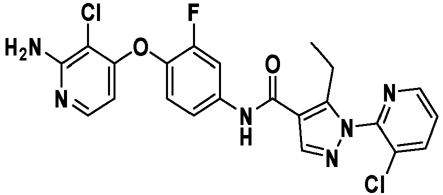 | N-(4-((2-amino-3-chloropyridin-4-yl)oxy)-3-fluorophenyl)-1-(3-chloropyridin-2-yl)-5-ethyl-1H-pyrazole-4-carboxamide |
| **Example37** | 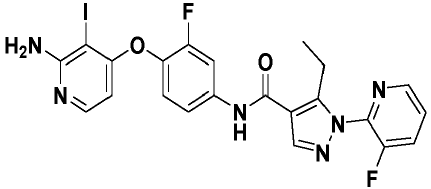 | N-(4-((2-amino-3-iodopyridin-4-yl)oxy)-3-fluorophenyl)-5-ethyl-1-(3-fluoropyridin-2-yl)-1H-pyrazole-4-carboxamide |
| **Example38** | 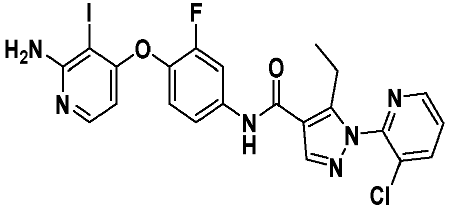 | N-(4-((2-amino-3-iodopyridin-4-yl)oxy)-3-fluorophenyl)-1-(3-chloropyridin-2-yl)-5-ethyl-1H-pyrazole-4-carboxamide |
| **Example39** | 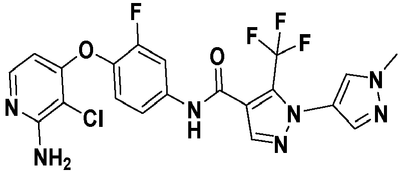 | N-(4-((2-amino-3-chloropyridin-4-yl)oxy)-3-fluorophenyl)-1'-methyl-5- (trifluoromethyl)-1'H-[1,4'-bipyrazole]-4-carboxamide |
| **Example40** | 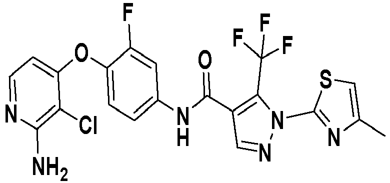 | N-(4-((2-amino-3-chloropyridin-4-yl)oxy)-3-fluorophenyl)-1-(4-methylthiazol-2-yl)-5-(trifluoromethyl)-1H-pyrazole-4-carboxamide |
| **Example41** | 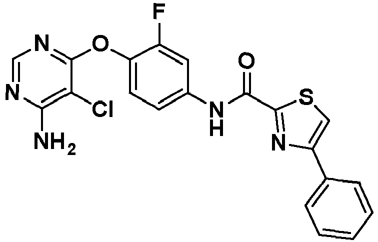 | N-[4-(6-amino-5-chloropyrimidin-4-yl)oxy-3-fluorophenyl]-4-phenyl-thiazole-2-carboxamide |
| **Example42** | 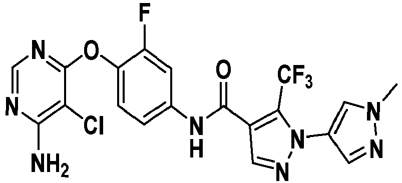 | N-[4-(6-amino-5-chloropyrimidin-4-yl)oxy-3-fluorophenyl]-1-(1-methylpyrazol-4-yl)-5-(trifluoromethyl)pyrazole-4-carboxamide |
| **Example43** | 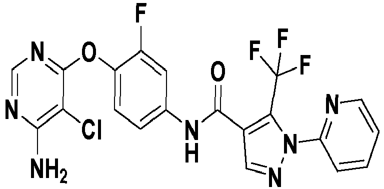 | N-(4-((6-amino-5-chloropyrimidin-4-yl)oxy)-3 - fluorophenyl)-1-(pyridin-2-yl)-5-(trifluoromethyl)-1H-pyrazole-4-carboxamide |
| **Example44** | 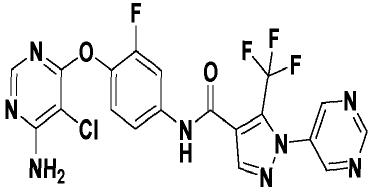 | N-[4-(6-amino-5-chloropyrimidin-4-yl)oxy-3-fluorophenyl]-1-pyrimidin-5-yl-5- (trifluoromethyl)pyrazole-4-carboxamide |
| **Example45** | 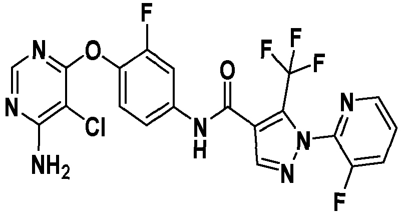 | N-[4-(6-amino-5-chloropyrimidin-4-yl)oxy-3-fluorophenyl]-1-(3-fluoro-2-pyridyl)-5-(trifluoromethyl)pyrazole-4-carboxamide |
| **Example46** | 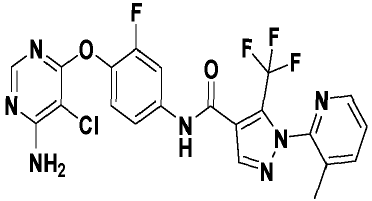 | N-[4-(6-amino-5-chloropyrimidin-4-yl)oxy-3-fluorophenyl]-1-(3-methyl-2-pyridyl)-5-(trifluoromethyl)pyrazole-4-carboxamide |
| **Example47** | 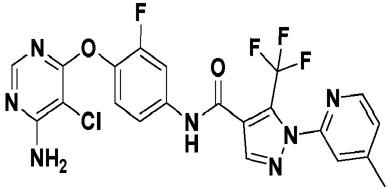 | N-[4-(6-amino-5-chloropyrimidin-4-yl)oxy-3-fluorophenyl]-1-(4-methyl-2-pyridyl)-5-(trifluoromethyl)pyrazole-4-carboxamide |
| **Example48** | 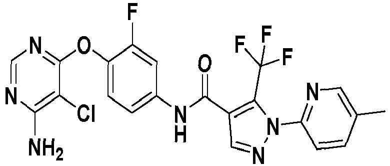 | N-[4-(6-amino-5-chloropyrimidin-4-yl)oxy-3-fluorophenyl]-1-(5-methyl-2-pyridyl)-5-(trifluoromethyl)pyrazole-4-carboxamide |
| **Example49** | 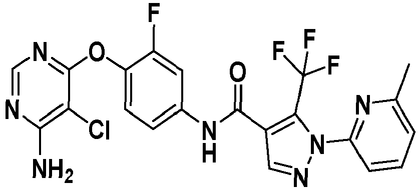 | N-[4-(6-amino-5-chloropyrimidin-4-yl)oxy-3-fluorophenyl]-1-(6-methyl-2-pyridyl)-5-(trifluoromethyl)pyrazole-4-carboxamide |
| **Example50** | 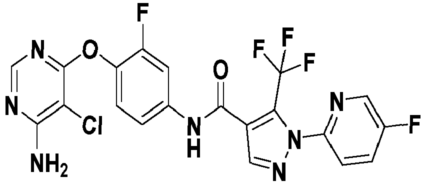 | N-[4-(6-amino-5-chloropyrimidin-4-yl)oxy-3-fluorophenyl]-1-(5-fluoro-2-pyridyl)-5-(trifluoromethyl)pyrazole-4-carboxamide |
| **Example51** | 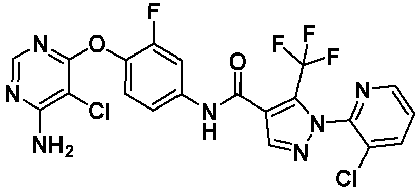 | N-[4-(6-amino-5-chloropyrimidin-4-yl)oxy-3-fluorophenyl]-1-(3-chloro-2-pyridyl)-5-(trifluoromethyl)pyrazole-4-carboxamide |
| **Example52** | 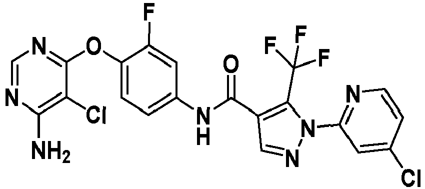 | N-[4-(6-amino-5-chloropyrimidin-4-yl)oxy-3-fluorophenyl]-1-(4-chloro-2-pyridyl)-5-(trifluoromethyl)pyrazole-4-carboxamide |
| **Example53** | 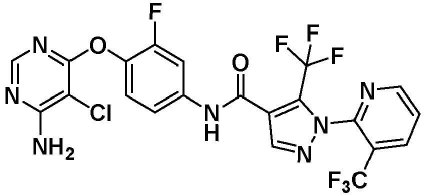 | N-[4-(6-amino-5-chloropyrimidin-4-yl)oxy-3-fluorophenyl]-5-(trifluoromethyl)-1-[3-(trifluoromethyl)-2-pyridyl]pyrazole-4-carboxamide |
| **Example54** | 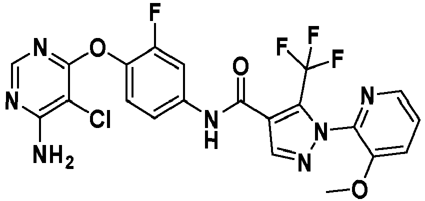 | N-[4-(6-amino-5-chloropyrimidin-4-yl)oxy-3-fluorophenyl]-1-(3-methoxy-2-pyridyl)-5-(trifluoromethyl)pyrazole-4-carboxamide |
| **Example55** | 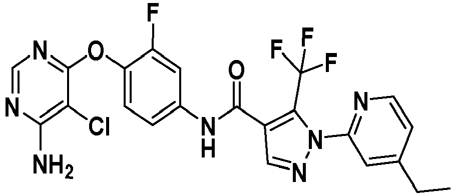 | N-[4-(6-amino-5-chloropyrimidin-4-yl)oxy-3-fluorophenyl]-1-(4-ethyl-2-pyridyl)-5-(trifluoromethyl)pyrazole-4-carboxamide |
| **Example56** | 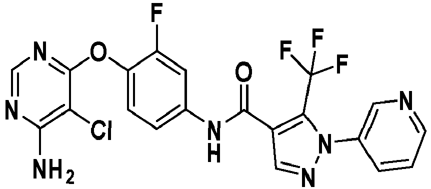 | N-[4-(6-amino-5-chloropyrimidin-4-yl)oxy-3-fluorophenyl]-1-(3-pyridyl)-5- (trifluoromethyl)pyrazole-4-carboxamide |
| **Example57** | 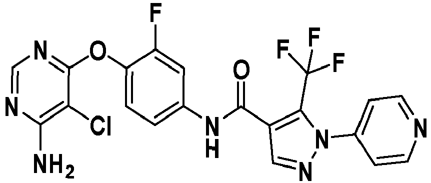 | N-[4-(6-amino-5-chloropyrimidin-4-yl)oxy-3-fluorophenyl]-1-(4-pyridyl)-5-(trifluoromethyl)pyrazole-4-carboxamide |
| **Example58** | 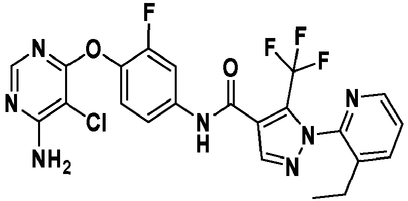 | N-[4-(6-amino-5-chloropyrimidin-4-yl)oxy-3-fluorophenyl]-1-(3-ethyl-2-pyridyl)-5-(trifluoromethyl)pyrazole-4-carboxamide |
| **Example59** | 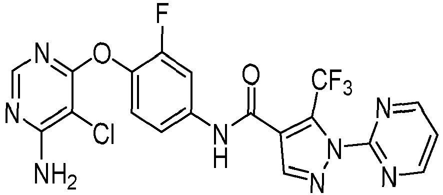 | N-[4-(6-amino-5-chloropyrimidin-4-yl)oxy-3-fluorophenyl]-1-pyrimidin-2-yl-5-(trifluoromethyl)pyrazole-4-carboxamide |
| **Example60** | 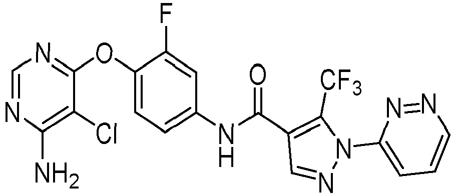 | N-[4-(6-amino-5-chloropyrimidin-4-yl)oxy-3-fluorophenyl]-1-pyridazin-3-yl-5- (trifluoromethyl)pyrazole-4-carboxamide |
| **Example61** | 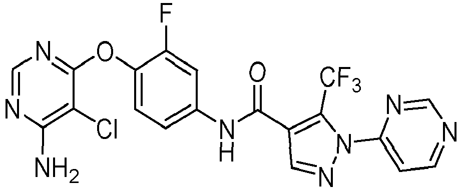 | N-[4-(6-amino-5-chloropyrimidin-4-yl)oxy-3-fluorophenyl]-1-pyrimidin-4-yl-5-(trifluoromethyl)pyrazole-4-carboxamide |
| **Example62** | 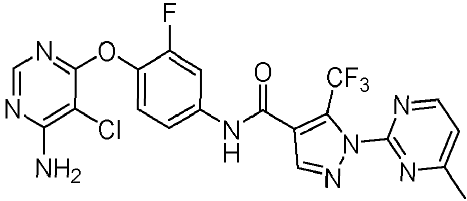 | N-[4-(6-amino-5-chloropyrimidin-4-yl)oxy-3-fluorophenyl]-1-(4-methylpyrimidin-2-yl)-5-(trifluoromethyl)pyrazole-4-carboxamide |
| **Example63** | 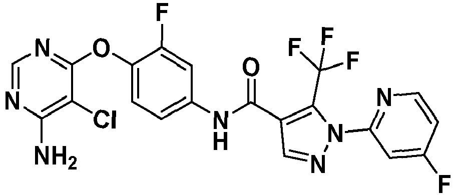 | N-[4-(6-amino-5-chloropyrimidin-4-yl)oxy-3-fluorophenyl]-1-(4-fluoro-2-pyridyl)-5-(trifluoromethyl)pyrazole-4-carboxamide |
| **Example64** | 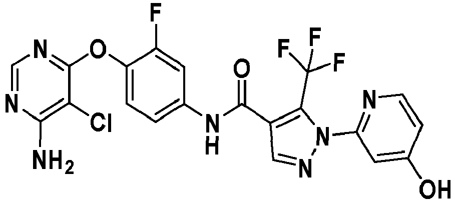 | N-[4-(6-amino-5-chloropyrimidin-4-yl)oxy-3-fluorophenyl]-1-(4-hydroxy-2-pyridyl)-5-(trifluoromethyl)pyrazole-4-carboxamide |
| **Example65** | 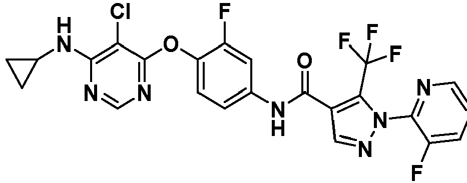 | N-[4-[5-chloro-6-(cyclopropylamino)pyrimidin-4-yl]oxy-3-fluoro-phenyl]-1-(3 -fluoro-2-pyridyl)-5 - (trifluoromethyl)pyrazole-4-carboxamide |
| **Example66** | 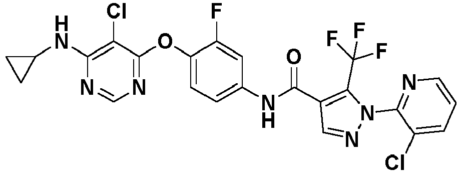 | N-[4-[5-chloro-6-(cyclopropylamino)pyrimidi n-4-yl]oxy-3-fluoro-phenyl]-1-(3-chloro-2-pyridyl)-5-(trifluoromethyl)pyrazole-4-carboxamide |

In the present disclosure, the "pharmaceutically acceptable salt" includes a salt commonly used to form an alkali metal salt and to form an additional salt of a free acid or a free base. The properties of these salts are not important, but should be pharmaceutically acceptable. A suitable pharmaceutically acceptable acid addition salt of the compound of Formula 1 may be prepared from an inorganic or organic acid. Examples of such an inorganic acid include hydrochloric acid, hydrobromic acid, hydroiodic acid, nitric acid, carbonic acid, sulfuric acid, and phosphoric acid. A suitable organic acid may be selected from aliphatic, alicyclic, aromatic, arylaliphatic, heterocyclic, carboxylic and sulfonic groups of organic acids, and examples of the organic acid include formic acid, acetic acid, adipic acid, butyric acid, propionic acid, succinic acid, glycolic acid, gluconic acid, lactic acid, malic acid, tartaric acid, citric acid, ascorbic acid, glucuronic acid, maleic acid, fumaric acid, pyruvic acid, aspartic acid, glutamic acid, benzoic acid, anthranilic acid, mesylic acid, 4-hydroxybenzoic acid, phenylacetic acid, mandelic acid, embonic acid (pamoic acid), methanesulfonic acid, ethanesulfonic acid, ethanedisulfonic acid, benzenesulfonic acid, pantothenic acid, 2-hydroxyethanesulfonic acid, toluenesulfonic acid, sulfanilic acid, cyclohexylaminosulfonic acid, camphoric acid, camphorsulfonic acid, digluconic acid, cyclopentanepropionic acid, dodecylsulfonic acid, glucoheptanoic acid, glycerophosphonic acid, heptanoic acid, hexanoic acid, 2-hydroxy-ethanesulfonic acid, nicotinic acid, 2-naphthalenesulfonic acid, oxalic acid, palmoic acid, pectic acid, persulfuric acid, 2-phenylpropionic acid, picric acid, pivalic acid, propionic acid, succinic acid, tartaric acid, thiocyanic acid, mesylic acid, undecanoic acid, stearic acid, algenic acid, β-hydroxybutyric acid, salicylic acid, galactaric acid, and galacturonic acid. A suitable pharmaceutically acceptable base addition salt of the compound of Formula 1 includes metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, aistidine, glucamine, isopropylamine, lysine, morpholine, N-ethyl morpholine, piperazine, piperidine, triethyl amine, and trimethyl amine. All of these salts may be prepared by conventional means from the corresponding compound of the invention by reacting, for example, the appropriate acid or base with the compound of Formula 1. When a basic group and an acid group are present in the same molecule, the compound of Formula 1 may also form internal salts.

In the present invention, the "solvate" may include a hydrate, and a solvate with an organic solvent such as methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and a mixture thereof.

According to another aspect, the present invention provides a pharmaceutical composition including a novel compound of Formula 1 defined above or a pharmaceutically acceptable salt thereof, and a pharmaceutically acceptable carrier.

The pharmaceutical composition may be useful for the prevention or treatment of protein kinase-mediated diseases, but the use of the present invention is not limited to these diseases.

In an embodiment, the pharmaceutical composition may be useful for the prevention or treatment of RON-mediated diseases.

The pharmaceutical composition according to the present invention includes a therapeutically effective amount of the compound of Formula 1 above or a pharmaceutically acceptable salt thereof.

In an embodiment, the disease may be cancer selected from the group consisting of lung cancer, breast cancer, colorectal cancer, kidney cancer, pancreatic cancer, head cancer, neck cancer, hereditary papillary renal cell carcinoma, pediatric hepatocellular carcinoma, and stomach cancer, but is not limited thereto.

In another embodiment, the disease may be an immune disease selected from the group consisting of an inflammatory disorder, a cardiovascular disease, a virus induced disease, a circulatory disease, a fibro-proliferative disease, and pain sensitization, but is not limited thereto.

For the treatment of the above-described diseases, the pharmaceutical composition may be administered to a subject in need of the prevention or treatment of the above-described diseases.

In the present specification, the term "prevention" refers to reducing the risk of developing a disease or disorder, and refers to any action that inhibits or delays the onset of a disease by preventing the progression of one or more clinical symptoms of the disease in a subject that is easily exposed to or susceptible to the disease but does not yet experience the disease or display symptoms of the disease.

In the present specification, the term "treatment" refers to alleviating a disease or disorder, and refers to any action that ameliorates or beneficially alters symptoms of the disease by arresting or reducing the progression of the disease or one or more clinical symptoms thereof.

In the present specification, the term "carrier" refers to a compound that facilitates the introduction of a compound into the cell or tissue. The pharmaceutical composition may be prepared in a unit dose form by being formulated using a pharmaceutically acceptable carrier, or may be prepared by being incorporated into a multidose container. In this case, the formulation may be in the form of a solution, a suspension, or an emulsion in an oil or an aqueous medium, or may be in the form of an extract, a powder, a granule, a tablet, a capsule, or a gel (e.g., a hydrogel), and may additionally include a dispersant or a stabilizer.

In addition, the compound represented by Formula 1 or the pharmaceutically acceptable salt thereof included in the pharmaceutical composition may be delivered in a pharmaceutically acceptable carrier such as colloidal suspensions, powders, saline, lipids, liposomes, microspheres, or nanospherical particles. These may be complexed with or associated with a vehicle and may be delivered in vivo using delivery systems known in the art, such as lipids, liposomes, microparticles, gold, nanoparticles, polymers, condensation reagents, polysaccharides, polyamino acids, dendrimers, saponins, adsorption enhancing substances, or fatty acids.

In addition, the pharmaceutically acceptable carrier may include lactose, dextrose, sucrose, sorbitol, mannitol, starch, acacia, rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil, and the like, which are typically used in formulation, but is not limited thereto. In addition, lubricants, wetting agents, sweetening agents, flavoring agents, emulsifying agents, suspending agents, preservatives, and the like may be further included in addition to the above components.

The pharmaceutical composition according to the present invention may be administered orally or parenterally at the time of clinical administration, and may be used in the form of a general pharmaceutical formulation. That is, the pharmaceutical composition of the present invention may be administered in various formulations of oral and parenteral administration at the time of actual clinical administration, and when formulated, is prepared using diluents or excipients such as fillers, extenders, binders, wetting agents, disintegrants, and surfactants, which are commonly used. Solid formulations for oral administration include tablets, pills, powders, granules, capsules, and the like, and such solid formulations are prepared by mixing a herbal medicine extract or a herbal medicine fermented product with at least one excipient, for example, starch, calcium carbonate, sucrose or lactose, gelatin, and the like. In addition to simple excipients, lubricants such as magnesium stearate and talc are also used. Liquid formulations for oral administration include suspensions, oral liquids, emulsions, syrups, and the like, and in addition to water and liquid paraffin, which are commonly used simple diluents, various excipients such as wetting agents, sweetening agents, fragrances, preservatives, and the like may be included. Formulations for parenteral administration include sterilized aqueous solutions, non-aqueous solvents, suspensions, emulsions, lyophilized formulations, and suppositories. For the non-aqueous solutions and the suspensions, propylene glycol, polyethylene glycol, vegetable oil such as olive oil, injectable ester such as ethyl oleate, or the like may be used. As the base of the suppositories, Witepsol^{™}, Macrogol, Tween^{™} 61, cacao butter, laurin fat, glycerol, gelatin, and the like may be used.

When the pharmaceutical composition is administered for a clinical purpose, the effective dosage of compound of Formula 1 above or the pharmaceutically acceptable salt thereof may vary depending on factors such as formulation methods, administration methods, patient's age, body weight, sex, condition, and diet, time of administration, route of administration, rate of excretion, drug combination, and sensitivity of response, but may generally be 0.01 mg/day to 20 mg/day per 1 kg body weight of an adult patient, preferably 1 mg/day to 10 mg/day, and may be administered in split doses several times a day, preferably 2 to 3 times a day at regular time intervals according to the decision of a doctor or a pharmacist.

In another aspect, the present invention provides a method for treating protein kinase-mediated diseases, in particular, RON-mediated diseases, the method including administering to a subject a therapeutically effective amount of the compound of Formula 1 above or the pharmaceutically acceptable salt thereof.

In another aspect, the present invention provides a method for inhibiting the activity of a RON receptor, the method including administering to a subject a therapeutically effective amount of the compound of Formula 1 above or the pharmaceutically acceptable salt thereof.

In the present specification, the term "therapeutically effective amount" refers to an amount of each formulation that achieves the purpose of reducing the severity of a disease and frequency of onset thereof during the treatment by each formulation itself, but avoids adverse reactions typically associated with other therapies. For example, an effective therapeutic agent for tumor exhibits effects of prolonging the survival period of a patient, suppressing the proliferation of cells associated with a tumor, or regressing the tumor.

Hereinafter, example compounds of the present invention may be prepared according to the following method, but compounds of the present invention are not limited to the preparation method.

### Preparation Example 1. 4-ethoxy-1-(4-fluorophenyl)-2-oxo-1,2-dihydropyridine-3-carboxylic acid

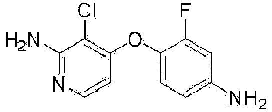

Hereinafter, a compound of Preparation Example 1 was prepared according to Reaction Scheme 1 below.

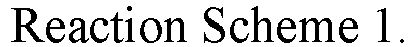

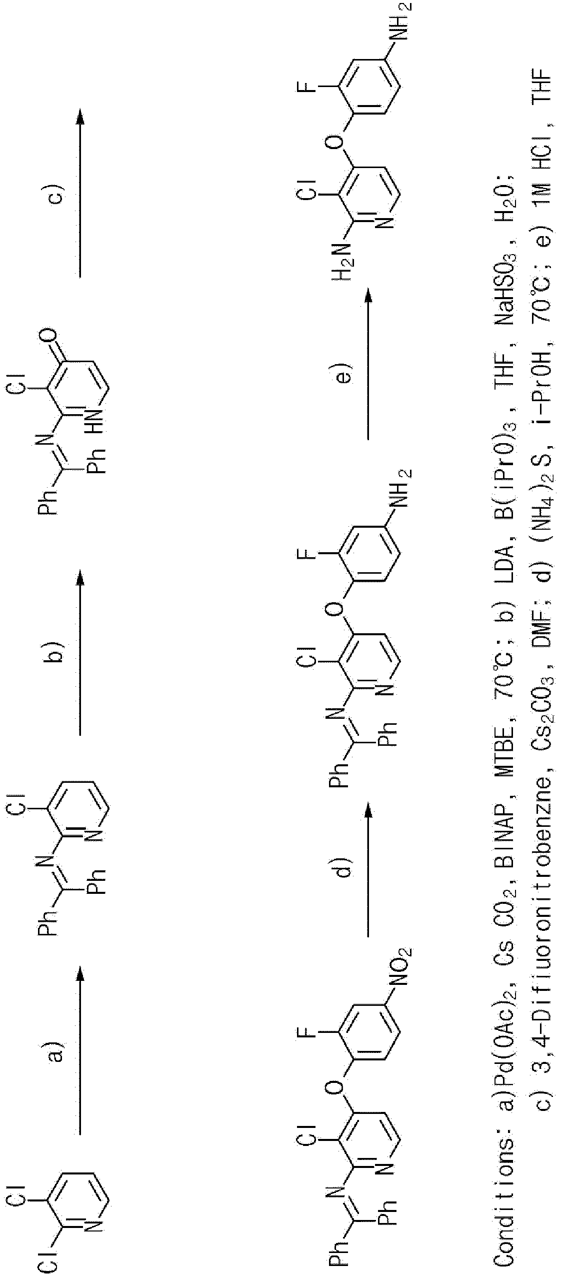

### Step 1) N-(3-chloropyridin-2-yl)-1,1-diphenylmethanimine

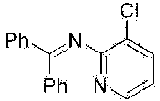

2,3-dichloropyridine (21 g, 142 mmol) was dissolved in methyl-tert-butyl ether (MTBE), and then Pd(OAc)2 (318 mg, 1.42 mmol), rac-BINAP (1.4 g, 2.13 mmol), Cs2CO3 (0.9 g, 2.84 mmol), and benzophenone imine (26 g, 142 mmol) were added thereto. The reaction mixture was stirred under reflux at 70 °C for 12 hours. The reaction mixture was cooled to room temperature, and the resulting solids were filtered with celite to concentrate the solvent. The residue was purified by column chromatography to obtain the title compound (20 g, yield: 48%).
MS m/z: 293 [M+H]

### Step 2) N-(3-chloropyridin-2-yl)-1,1-diphenylmethanimine

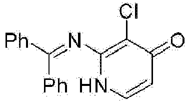

The compound N-(3-chloropyridin-2-yl)-1,1-diphenylmethanimine (20 g, 68.3 mmol) obtained in Step 1 above was dissolved in THF (100 ml) and tri-isopropyl borate (19.3 g, 102 mmol) was added thereto, followed by cooling to 0 °C. To the reaction mixture, lithium diisopropylamide (11 mL, 88.8 mmol) was slowly added at 0 °C. The reaction mixture was stirred at 0 °C for 2 hours, water (100 ml) was added thereto, and then sodium percarbonate (16 g, 102 mmol) was added thereto, followed by further stirring at room temperature for 3 hours. To the reaction mixture, saturated NaHCOs aqueous solution (50 ml) was slowly added and the mixture was extracted three times with ethyl acetate. The organic layer was concentrated and used in Step 3 below without a purification process.
MS m/z: 309 [M+H]

### Step 3) N-(3-chloro-4-(2-fluoro-4-nitrophenoxy)pyridin-2-yl-1,1-diphenylmethanimine

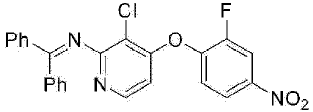

The compound N-(3-chloropyridin-2-yl)-1,1-diphenylmethanimine (20 g, 65 mmol) was dissolved in DMF, and then 3,4-difluoronitrobenzne (9.3 ml, 84.5 mmol) and Cs₂CO₃ (27.5 g, 84.5 mmol) were added thereto, followed by stirring at 90 °C for 1 hour. The reaction mixture was cooled to room temperature, diluted with water, and extracted three times with ethyl acetate. The organic solution was washed with brine and concentrated. The residue was purified by column chromatography to obtain a title compound (21 g, yield: 72%).
MS m/z: 448 [M+H]

### Step 4) 4-((3-chloro-2-(diphenylmethylene)amino)pyridin-4-yl)oxy-3-fluoroaniline

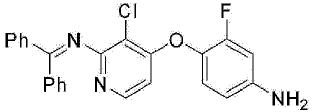

The compound N-(3-chloro-4-(2-fluoro-4-nitrophenoxy)pyridin-2-yl-1,1-diphenylmethanimine (20 g, 44.6 mmol) obtained in Step 3 above was dissolved in isopropanol, ammonium sulfide (30.4 ml, 446 mmol) was added thereto, followed by stirring at room temperature for 1 hour. Thereafter, the reaction mixture was stirred again at 70 °C for 3 hours. After the reaction was completed, water was added thereto, and the reaction mixture was cooled to room temperature. The resulting solids were obtained by filtering, butyl acetate was added thereto, and heated and dissolved at 80 °C, heptane was added thereto, and the reaction mixture was cooled to room temperature. After the reaction mixture was cooled to room temperature, the resulting solids were filtered and dried to obtain the title compound (14 g, 75%).
MS *m*/*z:* 418 [M+H]

### Step 5) 4-(4-amino-2-fluorophenoxy)-3-chloropyridin-2-amine

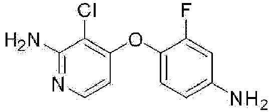

The compound 4-((3-chloro-2-(diphenylmethylene)amino)pyridine-4-yl)oxy-3-fluoroaniline (14 g, 33 mmol) obtained in Step 4 above was dissolved in THF, 1 M HCl (14 ml) was added thereto, followed by stirring at room temperature for 2 hours. The reaction mixture was concentrated, and then saturated NaHCOs was added thereto, and the reaction mixture was extracted three times with ethyl acetate. The organic solution was washed with brine and concentrated. The residue was purified by column chromatography to obtain the title compound (7.1 g, yield: 84%).
MS m/z: 254 [M+H]

### Preparation Example 2. 1-(3-methylpyridin-2-yl)-5-(trifluoromethyl)-1H-pyrazole-4-carboxylic acid

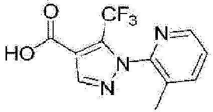

Hereinafter, a compound of Preparation Example 2 was prepared according to Reaction Scheme 2 below.

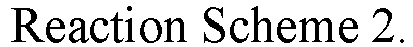

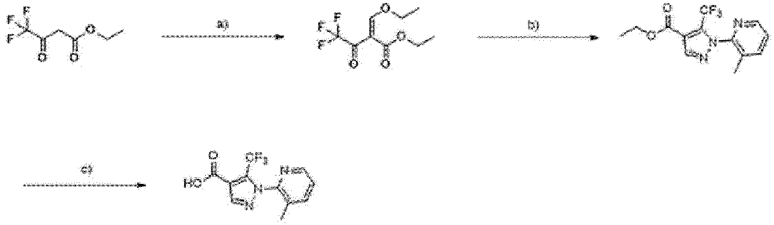

### Step 1) Ethyl 2-(ethoxymethylene)-4,4,4-trifluoro-3-oxobutanoate

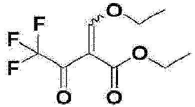

Ethyl 4,4,4-trifluoro-3-oxobutanoate (2.0 g, 10.9 mmol) and triethoxymethane (2.0 g, 14.1 mmol) were dissolved in acetic anhydride (3.3 g, 32.6 mmol), followed by stirring at 130°C for 4 hours. The reaction mixture was concentrated to obtain the title compound (2.0 g, yield: 77%).

MS m/z: 241[M+H].

### Step 2) Ethyl 1-(3-methylpyridin-2-yl)-5-(trifluoromethyl)-1H-pyrazole-4-carboxylate

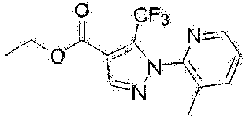

The compound ethyl 2-(ethoxymethylene)-4,4,4-trifluoro-3-oxobutanoate (0.5 g, 2.1 mmol) obtained in step 1 above and 2-hydrazinyl-4-methylpyridine (0.23 g, 1.9 mmol) were dissolved in 6 ml of ethanol, and then the reaction mixture was stirred at 60 °C for 12 hours, then extracted with ethyl acetate and water. The residue was purified by column chromatography to obtain the title compound (0.38 g, yield: 56%).
1H NMR (500 MHz, CDCl3) δ 8.46 (d, 1H), 8.20 (s, 1H), 7.77 (d, 1H), 7.43 (dd, 1H), 4.41 (q, 2H), 2.19 (s, 3H), 1.41 (t, 3H);
MS m/z: 300[M+H].

### Step 3) 1-(3-methylpyridin-2-yl)-5-(trifluoromethyl)-1H-pyrazole-4-carboxylic acid

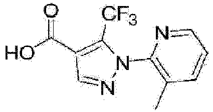

The compound ethyl 1-(3-methylpyridine-2-yl)-5-(trifluoromethyl)-1H-pyrazole-4-carboxylate (0.38 g, 1.3 mmol) obtained in step 2 above was dissolved in 4 ml of ethanol, and then 2 ml of a 6N sodium hydroxide solution was added thereto at room temperature, followed by stirring for 1 hour. Ice water was added to the reaction product, and the resulting solid was filtered to obtain the title compound (0.31 g, yield: 90%).
¹H NMR (500 MHz, DMSO-d₆) δ 13.42 (brs, 1H), 8.46 (d, 1H), 8.31 (s, 1H), 8.02 (d, 1H), 7.62 (dd, 1H), 2.12 (s, 3H);
MS *m*/*z*: 272[M+H].

### Example 1. N-(4-((2-amino-3-chloropyridin-4-yl)oxy)-3-fluorophenyl)-5-ethyl-1-(pyrimidin-5-yl)-1H-pyrazole-4-carboxamide

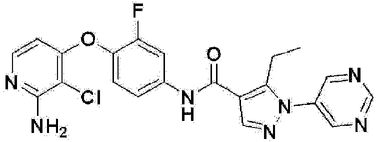

For the compound of Example 1, the compound 4-(4-amino-2-fluorophenoxy)-3-chloropyridine-2-amine (50 mg, 0.20 mmol) obtained in Preparation Example 1 was dissolved in 3 ml of THF, and then 5-ethyl-1-(pyrimidin-5-yl)-1H-pyrazole-4-carboxylic acid (61 mg, 0.24 mmol), HATU (114 mg, 0.3 mmol), and DIPEA (52 µl, 0.3 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (43 mg, yield: 48%).
¹H NMR (400 MHz, MeOH-d₄) δ 9.33 (s, 1H), 9.06 (s, 2H), 8.34 (s, 1H), 7.90 (d, 1H), 7.75 (d, 1H), 7.53 (m, 1H), 7.27 (t, 1H), 6.07 (d, 1H), 3.14 (q, 2H), 1.25 (t, 3H); MS *m*/*z:* 454[M+H]

### Example 2. N-(4-((2-amino-3-chloropyridin-4-yl)oxy)-3-fluorophenyl)-1-(pyridin-2-yl)-5-(trifluoromethyl)-1H-pyrazole-4-carboxamide

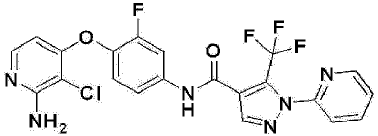

The title compound (59 mg, yield: 76%) was obtained in a manner similar to that of Example 1 using 1-(pyridin-2-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.88 (s, 1H), 8.63 (d, 1H), 8.38 (s, 1H), 8.16 (t, 1H), 7.91 (d, 1H), 7.88 (m, 1H), 7.79 (d, 1H), 7.65 (m, 1H), 7.52 (m, 1H), 7.38 (m, 1H), 6.48 (brs, 2H), 5.98 (d, 1H); MS *m*/*z:* 493[M+H]

### Example 3. N-(4-((2-amino-3-chloropyridin-4-yl)oxy)-3-fluorophenyl)-1-(4-phenylthiazol-2-yl)-5-(trifluoromethyl)-1H-pyrazole-4-carboxamide

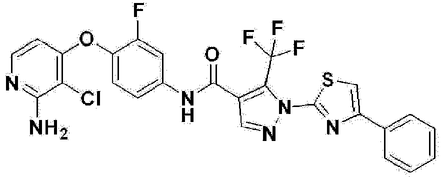

The title compound (46 mg, yield: 51%) was obtained in a manner similar to that of Example 1 using 1-(4-phenylthiazol-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 11.04 (s, 1H), 8.47 (s, 1H), 8.23 (s, 1H), 7.99 (m, 2H), 7.89 (d, 1H), 7.78 (m, 1H), 7.53 (m, 3H), 7.43 (m, 2H), 6.44 (brs, 2H), 5.97 (d, 1H); MS *m*/*z:* 575[M+H]

### Example 4. N-(4-((2-amino-3-chloropyridin-4-yl)oxy)-3-fluorophenyl)-5-ethyl-1-(pyridin-4-yl)-1H-pyrazole-4-carboxamide

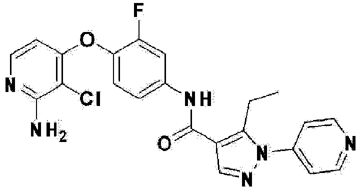

The title compound (31 mg, yield: 19%) was obtained in a manner similar to that of Example 1 using 5-ethyl-1-(pyridin-4-yl)-1H-pyrazole-4-carboxylic acid instead of 5-ethyl-1-(pyrimidin-5-yl)-1H-pyrazole-4-carboxylic acid according to Example 1.
MS *m*/*z*: 452[M+H]

### Example 5. N-(4-((2-amino-3-chloropyridin-4-yl)oxy)-3-fluorophenyl)-5-ethyl-1-(pyridin-3-yl)-1H-pyrazole-4-carboxamide

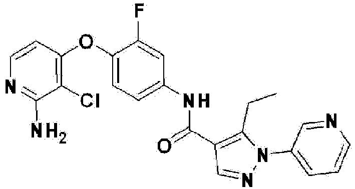

The title compound (16 mg, yield: 10%) was obtained in a manner similar to that of Example 1 using 5-ethyl-1-(pyridin-3-yl)-1H-pyrazole-4-carboxylic acid instead of 5-ethyl-1-(pyrimidin-5-yl)-1H-pyrazole-4-carboxylic acid according to Example 1.
MS *m*/*z*: 452[M+H]

### Example 6. N-(4-((2-amino-3-chloropyridin-4-yl)oxy)-3-fluorophenyl)-1-(pyrimidin-5-yl)-5-(trifluoromethyl)-1H-pyrazole-4-carboxamide

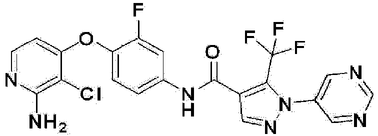

The title compound (45 mg, yield: 58%) was obtained in a manner similar to that of Example 1 using 1-(pyrimidin-5-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.84 (s, 1H), 9.41 (s,1H), 9.15 (s, 2H), 8.47 (s, 1H), 7.84 (d, 1H), 7.73 (s, 1H), 7.50 (d, 1H), 7.36 (m, 1H), 6.39 (brs, 2H), 5.92 (d, 1H); MS *m*/*z:* 494[M+H]

### Example 7. N-(4-((2-amino-3-chloropyridin-4-yl)oxy)-3-fluorophenyl)-5-methyl-1-(pyrimidin-5-yl)-1H-pyrazole-4-carboxamide

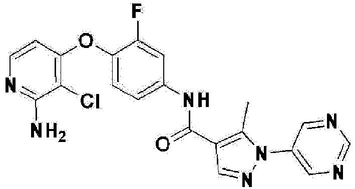

The title compound (35 mg, yield: 40%) was obtained in a manner similar to that of Example 1 using 5-methyl-1-(pyrimidin-5-yl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.18 (s, 1H), 9.29 (s, 1H), 9.11 (s, 2H), 8.41 (s, 1H), 7.90 (dd, 1H), 7.73 (d, 1H), 7.53 (dd, 1H), 7.31 (t, 1H), 6.38 (s, 2H), 5.91 (d, 1H); MS *m*/*z:* 440[M+H]

### Example 8. N-(4-((2-amino-3-chloropyridin-4-yl)oxy)-3-fluorophenyl)-1-(3-fluoropyridin-2-yl)-5-(trifluoromethyl)-1H-pyrazole-4-carboxamide

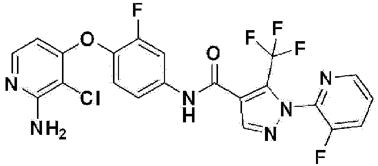

The title compound (20 mg, yield: 25%) was obtained in a manner similar to that of Example 1 using 1-(3-fluoropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.90 (s, 1H), 8.56 (d, 1H), 8.49 (s, 1H), 8.23 (t, 1H), 7.91 (m, 2H), 7.77 (d, 1H), 7.54 (d, 1H), 7.39 (t, 1H), 6.46 (s, 2H), 5.97 (d, 1H); MS *m*/*z:* 511[M+H]

### Example 9. N-(4-((2-amino-3-chloropyridin-4-yl)oxy)-3-fluorophenyl)-1-(3-methylpyridin-2-yl)-5-(trifluoromethyl)-1H-pyrazole-4-carboxamide

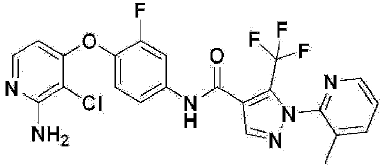

The title compound (20 mg, yield: 25%) was obtained in a manner similar to that of Example 1 using 1-(3-methylpyridin-2-yl)-5-(trifluoromethyl)-1H-pyrazole-4-carboxylic acid obtained in Preparation Example 2 instead of 5-ethyl-1-(pyrimidin-5-yl)-1H-pyrazole-4-carboxylic acid according to Example 1.
¹H NMR (400 MHz, DMSO-d₆) δ 10.84 (s, 1H), 8.48 (d, 1H), 8.42 (s, 1H), 8.04 (d, 1H), 7.91 (dd, 1H), 7.77 (d, 1H), 7.63 (dd, 1H), 7.55 (d, 1H), 7.38 (t, 1H), 6.44 (s, 2H), 5.96 (d, 1H), 2.17 (s, 3H); MS *m*/*z:* 507[M+H]

### Example 10. N-(4-((2-amino-3-chloropyridin-4-yl)oxy)-3-fluorophenyl)-1-(3-chloropyridin-2-yl)-5-(trifluoromethyl)-1H-pyrazole-4-carboxamide

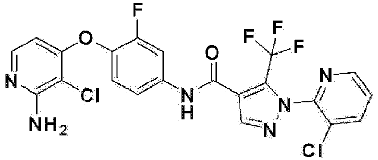

The title compound (23 mg, yield: 28%) was obtained in a manner similar to that of Example 1 using 1-(3-chloropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.87 (s, 1H), 8.67 (d, 1H), 8.50 (s, 1H), 8.41 (d, 1H), 7.91 (d, 1H), 7.82 (dd, 1H), 7.77 (d, 1H), 7.55 (d, 1H), 7.38 (t, 1H) 6.44 (s, 2H), 5.96 (d, 1H); MS *m*/*z:* 528[M+H]

### Example 11. N-(4-((2-amino-3-chloropyridin-4-yl)oxy)-3-fluorophenyl)-5-(trifluoromethyl)-1-(3-(trifluoromethyl)pyridin-2-yl)-1H-pyrazole-4-carboxamide

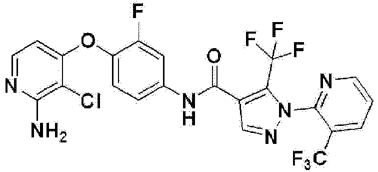

The title compound (25 mg, yield: 28%) was obtained in a manner similar to that of Example 1 using 1-(3-trifluoromethyl)pyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ10.88 (s, 1H), 8.99 (d, 1H), 8.67 (d, 1H), 8.50 (s, 1H), 8.02 (dd, 1H), 7.91 (dd, 1H), 7.77 (d, 1H), 7.57 (d, 1H), 7.38 (t, 1H), 6.44 (s, 2H), 5.96 (d, 1H); MS *m*/*z:* 561[M+H]

### Example 12. N-(4-((2-amino-3-chloropyridin-4-yl)oxy)-3-fluorophenyl)-1-(4-methylpyridin-2-yl)-5-(trifluoromethyl)-1H-pyrazole-4-carboxamide

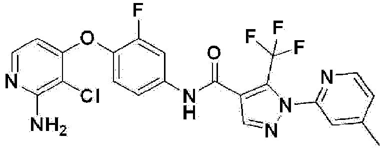

The title compound (27 mg, yield: 41%) was obtained in a manner similar to that of Example 1 using 1-(4-methylpyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.89 (s, 1H), 8.47 (d, 1H), 8.36 (s, 1H), 7.89 (d, 1H), 7.77 (d, 1H), 7.68 (s, 1H), 7.53 (d, 1H), 7.47 (d, 1H), 7.38 (t, 1H), 6.44 (s, 2H), 5.96 (d, 1H); MS *m*/*z:* 507[M+H]

### Example 13. N-(4-((2-amino-3-chloropyridin-4-yl)oxy)-3-fluorophenyl)-1-(5-fluoropyridin-2-yl)-5-(trifluoromethyl)-1H-pyrazole-4-carboxamide

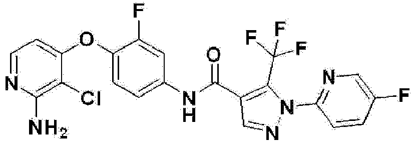

The title compound (20 mg, yield: 33%) was obtained in a manner similar to that of Example 1 using 1-(5-fluoropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.90 (s, 1H), 8.68 (d, 1H), 8.39 (s, 1H), 8.13 (m, 1H), 7.94 (m, 1H), 7.89 (m, 1H), 7.77 (d, 1H), 7.52 (d, 1H), 7.38 (t, 1H), 6.44 (s, 2H), 5.96 (d, 1H); MS *m*/*z:* 511[M+H]

### Example 14. N-(4-((2-amino-3-chloropyridin-4-yl)oxy)-3-fluorophenyl)-1-(4-chloropyridin-2-yl)-5-(trifluoromethyl)-1H-pyrazole-4-carboxamide

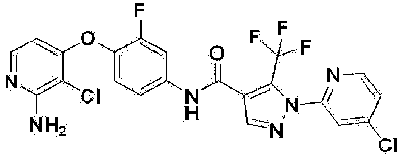

The title compound (20 mg, yield: 32%) was obtained in a manner similar to that of Example 1 using 1-(4-chloropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.96 (s, 1H), 8.92 (d, 1H), 8.46 (s, 1H), 8.25 (s, 1H), 8.06 (d, 1H), 7.90 (d, 1H), 7.77 (d, 1H), 7.53 (d, 1H), 7.53 (d, 1H), 7.38 (t, 1H), 6.45 (s, 2H), 5.96 (d, 1H); MS *m*/*z:* 528[M+H]

### Example 15. N-(4-((2-amino-3-chloropyridin-4-yl)oxy)-3-fluorophenyl)-1-(3-methoxypyridin-2-yl)-5-(trifluoromethyl)-1H-pyrazole-4-carboxamide

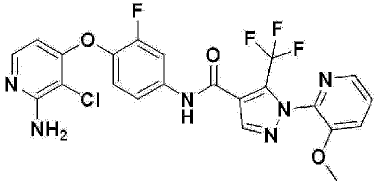

The title compound (25 mg, yield: 40%) was obtained in a manner similar to that of Example 1 using 1-(3-methoxypyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.76 (s, 1H), 8.34 (s, 1H), 8.17 (dd, 1H), 7.88-7.81 (m, 2H), 7.72 (d, 1H), 7.68 (dd, 1H), 7.51 (d, 1H), 7.33 (t, 1H), 6.40 (s, 2H), 5.91 (d, 1H), 3.82 (s, 3H); MS *m*/*z:* 523[M+H]

### Example 16. N-(4-((2-amino-3-chloropyridin-4-yl)oxy)-3-fluorophenyl)-1-(5-methylpyridin-2-yl)-5-(trifluoromethyl)-1H-pyrazole-4-carboxamide

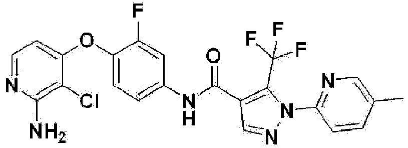

The title compound (24 mg, yield: 40%) was obtained in a manner similar to that of Example 1 using 1-(5-methylpyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.83 (s, 1H), 8.41 (s, 1H), 8.31 (s, 1H), 7.92 (d, 1H), 7.85 (d, 1H), 7.72 (d, 1H), 7.67 (d, 1H), 7.48 (d, 1H), 7.33 (t, 1H), 6.40 (s, 2H), 5.91 (d, 1H), 2.37 (s, 3H); MS *m*/*z:* 507[M+H]

### Example 17. N-(4-((2-amino-3-chloropyridin-4-yl)oxy)-3-fluorophenyl)-1-(6-methylpyridin-2-yl)-5-(trifluoromethyl)-1H-pyrazole-4-carboxamide

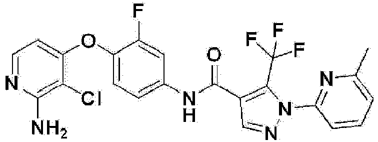

The title compound (3.5 mg, yield: 36%) was obtained in a manner similar to that of Example 1 using 1-(6-methylpyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹HNMR (400 MHz, DMSO-d₆) δ 10.84 (s, 1H), 8.30 (s, 1H), 7.98 (t, 1H), 7.85 (dd, 1H), 7.72 (d, 1H), 7.58 (d, 1H), 7.49-7.44 (m, 2H), 7.33 (t, 1H), 6.40 (s, 2H), 5.91 (d, 1H), 2.50 (s, 3H); MS *m*/*z:* 507[M+H]

### Example 18. N-(4-((2-amino-3-chloropyridin-4-yl)oxy)-3-fluorophenyl)-1-(pyridin-3-yl)-5-(trifluoromethyl)-1H-pyrazole-4-carboxamide

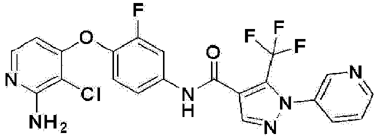

The title compound (25 mg, yield: 43%) was obtained in a manner similar to that of Example 1 using 1-(pyridin-3-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.88 (s, 1H), 8.84-8.81 (m, 2H), 8.43 (s, 1H), 8.10 (d, 1H), 7.90 (dd, 1H), 7.77 (d, 1H), 7.70 (dd, 1H), 7.54 (d, 1H), 7.39 (t, 1H), 6.50 (s, 2H), 5.97 (d, 1H); MS *m*/*z:* 493[M+H]

### Example 19. N-(4-((2-amino-3-chloropyridin-4-yl)oxy)-3-fluorophenyl)-1-(pyridin-4-yl)-5-(trifluoromethyl)-1H-pyrazole-4-carboxamide

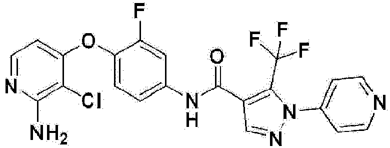

The title compound (6 mg, yield: 10%) was obtained in a manner similar to that of Example 1 using 1-(pyridin-4-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ10.90 (s, 1H), 8.87 (d, 1H), 8.46 (s, 1H), 7.89 (d, 1H), 7.77 (d, 1H), 7.68 (d, 1H), 7.53 (d, 1H), 7.38 (t, 1H), 6.45 (s, 2H), 5.96 (d, 1H); MS *m*/*z:* 493[M+H]

### Example 20. N-(4-((2-amino-3-chloropyridin-4-yl)oxy)-3-fluorophenyl)-1-(pyridazin-3-yl)-5-(trifluoromethyl)-1H-pyrazole-4-carboxamide

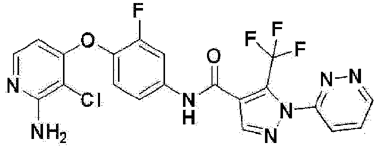

The title compound (3.5 mg, yield: 36%) was obtained in a manner similar to that of Example 1 using 1-(pyridazin-3-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.95 (s, 1H), 9.47 (d, 1H), 8.52 (s, 1H), 8.24 (d, 1H), 8.11 (dd, 1H), 7.90 (d, 1H), 7.77 (d, 1H), 7.54 (d, 1H), 7.39 (t, 1H), 6.49 (s, 2H), 5.98 (d, 1H); MS *m*/*z:* 494[M+H]

### Example 21. N-(4-((2-amino-3-chloropyridin-4-yl)oxy)-3-fluorophenyl)-1-(pyrimidin-2-yl)-5-(trifluoromethyl)-1H-pyrazole-4-carboxamide

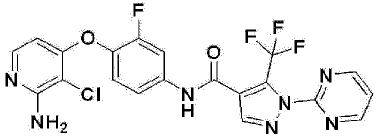

The title compound (10 mg, yield: 13%) was obtained in a manner similar to that of Example 1 using 1-(pyrimidin-2-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.88 (s, 1H), 9.05 (d, 1H), 8.38 (s, 1H), 7.85 (d, 1H), 7.78-7.73 (m, 2H), 7.49 (d, 1H), 7.34 (t, 1H), 6.51 (s, 1H), 5.95 (d, 1H); MS *m*/*z:* 494[M+H]

### Example 22. N-(4-((2-amino-3-chloropyridin-4-yl)oxy)-3-fluorophenyl)-1-(pyrimidin-4-yl)-5-(trifluoromethyl)-1H-pyrazole-4-carboxamide

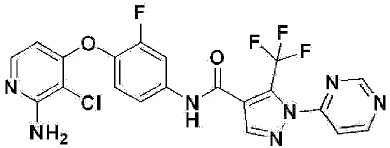

The title compound (10 mg, yield: 13%) was obtained in a manner similar to that of Example 1 using 1-(pyrimidin-4-yl)-5-(trifluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.95 (s, 1H), 9.24 (s, 1H), 9.07 (d, 1H), 8.45 (s, 1H), 8.02 (dd, 1H), 7.83 (dd, 1H), 7.73 (d, 1H), 7.46 (d, 1H), 7.34 (t, 1H), 6.45 (s, 2H), 5.93 (d, 1H); MS *m*/*z:* 494[M+H]

### Example 23. N-(4-((2-amino-3-chloropyridin-4-yl)oxy)-3-fluorophenyl)-1-(4-methylpyrimidin-2-yl)-5-(trifluoromethyl)-1H-pyrazole-4-carboxamide

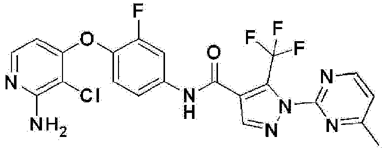

The title compound (20 mg, yield: 54%) was obtained in a manner similar to that of Example 1 using 1-(4-methylpyrimidin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
MS *m*/*z*: 508[M+H]

### Example 24. N-(4-((2-amino-3-chloropyridin-4-yl)oxy)-3-fluorophenyl)-1-(3-ethoxypyridin-2-yl)-5-(trifluoromethyl)-1H-pyrazole-4-carboxamide

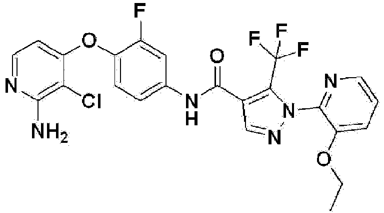

The title compound (20 mg, yield: 24%) was obtained in a manner similar to that of Example 1 using 1-(3-ethoxypyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (400 MHz, DMSO-d₆) δ 10.78 (s, 1H), 8.40 (s, 1H), 8.19 (d, 1H), 7.91 (d, 1H), 7.85 (d, 1H), 7.77 (d, 1H),7.68 (dd, 1H), 7.56 (d, 1H), 7.37 (t, 1H), 6.43 (s, 2H), 5.96 (d, 1H), 4.19 (q, 2H), 1.22 (t, 3H); MS *m*/*z:* 537[M+H]

### Example 25. N-(4-((2-amino-3-chloropyridin-4-yl)oxy)-3-fluorophenyl)-1-(4-fluoropyridin-2-yl)-5-(trifluoromethyl)-1H-pyrazole-4-carboxamide

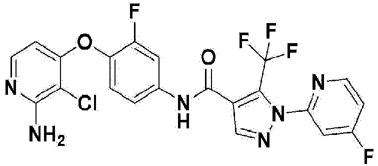

The title compound (30 mg, yield: 50%) was obtained in a manner similar to that of Example 1 using 1-(4-fluoropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.92 (s, 1H), 8.68 (d, 1H), 8.41 (s, 1H), 7.87 (m, 2H), 7.77 (d, 1H), 7.62 (t, 1H), 7.52 (d, 1H), 7.38 (t, 1H), 6.46 (s, 2H), 5.97 (d, 1H) ; MS *m*/*z:* 511[M+H]

### Example 26. N-(4-((2-amino-3-chloropyridin-4-yl)oxy)-3-fluorophenyl)-1-(5-fluoropyrimidin-2-yl)-5-(trifluoromethyl)-1H-pyrazole-4-carboxamide

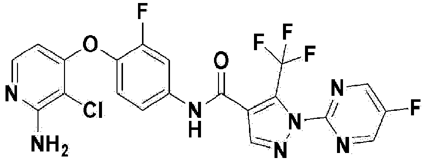

The title compound (28 mg, yield: 35%) was obtained in a manner similar to that of Example 1 using 1-(5-fluoropyrimidin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.93 (s, 1H), 9.20 (s, 2H), 8.43 (s, 1H), 7.92 (d, 1H), 7.79 (m, 1H), 7.55 (m, 1H), 7.42 (t, 1H), 6.76 (brs, 2H), 6.60 (m, 1H); MS *m*/*z:* 512[M+H]

### Example 27. N-(4-((2-amino-3-chloropyridin-4-yl)oxy)-3-fluorophenyl)-1-(5-hydroxypyrimidin-2-yl)-5-(trifluoromethyl)-1H-pyrazole-4-carboxamide

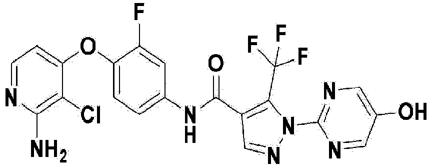

The title compound (10 mg, yield: 13%) was obtained in a manner similar to that of Example 1 using 1-(5-hydroxypyrimidin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.84 (s, 1H), 8.49 (s, 2H), 8.34 (s, 1H), 7.91 (d, 1H), 7.77 (d, 1H), 7.55 (m, 1H), 7.37 (t, 1H), 6.42 (brs, 2H), 5.97 (m, 1H); MS *m*/*z:* 510[M+H]

### Example 28. N-(4-((2-amino-3-iodopyridin-4-yl)oxy)-3-fluorophenyl)-1-(3-fluoropyridin-2-yl)-5-(trifluoromethyl)-1H-pyrazole-4-carboxamide

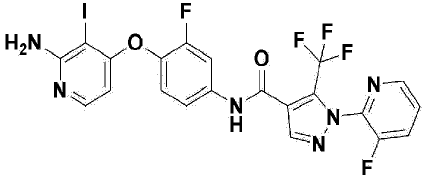

The compound 4-(4-amino-2-fluorophenoxy)-3-iodopyridine-2-amine (200 mg, 0.58 mmol) was dissolved in 4 ml of DMF, and then 1-(3-fluoropyridin-2-yl)-5-(trifluoromethyl)-1H-pyrazole-4-carboxylic acid (191 mg, 0.70 mmol), HATU (331 mg, 0.3 mmol), and DIPEA (202 µl, 0.3 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (300 mg, yield: 86%).

¹H NMR (500 MHz, DMSO-d₆) δ 10.89 (s, 1H), 8.55 (s, 1H), 8.49 (s, 1H), 8.25 (t, 1H), 7.90 (m, 2H), 7.75 (d, 1H), 7.54 (d, 1H), 7.34 (t, 1H), 6.22 (brs, 2H), 5.83 (d, 1H).

### Example 29. N-(4-((2-amino-3-chloropyridin-4-yl)oxy)-3-fluorophenyl)-1-(3,5-difluoropyridin-2-yl)-5-(trifluoromethyl)-1H-pyrazole-4-carboxamide

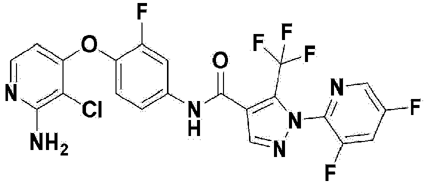

The title compound (21 mg, yield: 25%) was obtained in a manner similar to that of Example 1 using 1-(3 , 5-difluoropyridin-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.87 (s, 1H), 8.64 (s, 1H), 8.47 (m, 2H), 7.87 (d, 1H), 7.73 (s, 1H), 7.52 (d, 1H), 7.33 (t, 1H), 6.38 (brs, 2H), 5.91 (d, 1H); MS *m*/*z:* 529[M+H]

### Example 30. N-(4-((2-amino-3-chloropyridin-4-yl)oxy)-3-fluorophenyl)-1-(6-fluoropyridazin-3-yl)-5-(trifluoromethyl)-1H-pyrazole-4-carboxamide

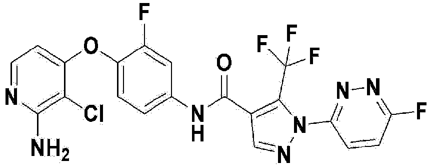

The title compound (22 mg, yield: 27%) was obtained in a manner similar to that of Example 1 using 1-(6-fluoropyridazin-3-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.83 (s, 1H), 8.38 (s, 1H), 7.81 (m, 2H), 7.71 (s, 1H), 7.50 (d, 1H), 7.37 (m, 1H), 7.14 (d, 1H) 6.38 (brs, 2H), 5.92 (d, 1H); MS *m*/*z:* 512[M+H]

### Example 31. N-(4-((2-aminopyridin-4-yl)oxy)-3-fluorophenyl)-1-(3-fluoropyridin-2-yl)-5-(trifluoromethyl)-1H-pyrazole-4-carboxamide

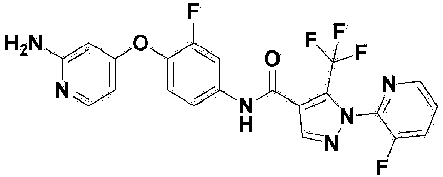

The title compound (3 mg, yield: 8%) was obtained in a manner similar to that of Example 8 using 4-(4-amino-2-fluorophenoxy)pyridine-2-amine instead of 4-(4-amino-2-fluorophenoxy)-3-chloropyridine-2-amine according to Example 1.

¹H NMR (500 MHz, DMSO-d₆) δ 10.89 (s, 1H), 8.55 (s, 1H), 8.48 (d, 1H), 8.23 (t, 1H), 7.82 (m, 3H), 7.54 (d, 1H), 7.34 (t, 1H), 6.19 (d, 1H), 5.96 (s, 2H).

### Example 32. N-(4-((2-amino-3-chloropyridin-4-yl)oxy)-2-fluorophenyl)-1-(3-fluoropyridin-2-yl)-5-(trifluoromethyl)-1H-pyrazole-4-carboxamide

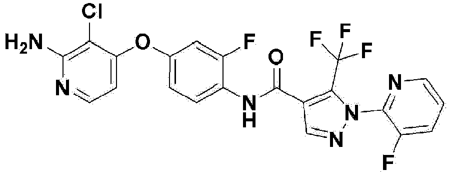

The title compound (19 mg, yield: 50%) was obtained in a manner similar to that of Example 8 using 4-(4-amino-3-fluorophenoxy)-3-chloropyridine-2-amine instead of 4-(4-amino-2-fluorophenoxy)-3-chloropyridine-2-amine according to Example 1.
¹H NMR (500 MHz, DMSO-d₆) δ 10.51 (s, 1H), 8.56 (s, 1H), 8.48 (d, 1H), 8.26 (t, 1H), 7.86 (m, 2H), 7.77 (m, 1H), 7.26 (m, 1H), 7.02 (m, 1H), 6.46 (brs, 2H), 6.13 (d, 1H); MS *m*/*z:* 511[M+H]

### Example 33. N-(4-((2-amino-3-(cyclohexylamino)pyridin-4-yl)oxy)-3-fluorophenyl)-1-(3-fluoropyridin-2-yl)-5-(trifluoromethyl)-1H-pyrazole-4-carboxamide

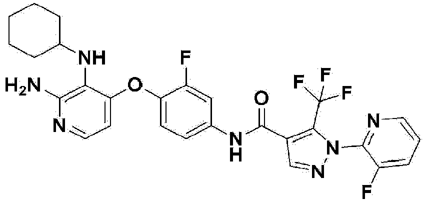

The compound 4-(4-amino-2-fluorophenoxy)-N3-cyclohexylpyridine-2,3-diamine (20 mg, 0.06 mmol) was dissolved in 1 ml of DMF, and then 1-(3-fluoropyridin-2-yl)-5-(trifluoromethyl)-1H-pyrazole-4-carboxylic acid (19 mg, 0.07 mmol), HATU (33 mg, 0.3 mmol), and DIPEA (20 µl, 0.11 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (29 mg, yield: 89%).

¹H NMR (500 MHz, DMSO-d₆) δ 10.83 (s, 1H), 8.56 (d, 1H), 8.48 (s, 1H), 8.23 (t, 1H), 7.86 (t, 1H), 7.87 (m, 2H), 7.49 (m, 1H), 7.18 (t, 1H), 5.92 (d, 1H), 5.66 (brs, 2H), 3.07 (m, 1H), 1.91 (m, 2H), 1.84 (m, 1H), 1.20 (m, 6H).

### Example 34. N-(4-((2-amino-3-(isopropylamino)pyridin-4-yl)oxy)-3-fluorophenyl)-1-(3-fluoropyridin-2-yl)-5-(trifluoromethyl)-1H-pyrazole-4-carboxamide

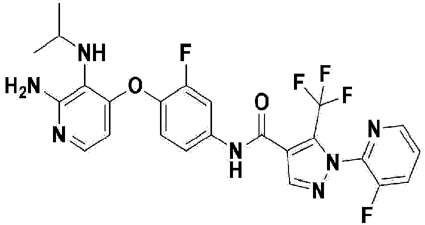

The compound 4-(4-amino-2-fluorophenoxy)-N3-isopropylpyridine-2,3-diamine (25 mg, 0.08 mmol) was dissolved in 1 ml of DMF, and then 1-(3-fluoropyridin-2-yl)-5-(trifluoromethyl)-1H-pyrazole-4-carboxylic acid (24 mg, 0.09 mmol), HATU (37 mg, 0.3 mmol), and DIPEA (56 µl, 0.32 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (9 mg, yield: 21%).

¹H NMR (500 MHz, MeOD-d4) δ 8.50 (d, 1H), 8.30 (s, 1H), 8.01 (m, 1H), 7.87 (m, 1H), 7.78 (m, 1H), 7.54 (m, 1H), 7.49 (m, 1H), 7.24 (t, 1H), 6.05 (d, 1H), 3.54 (m, 1H), 1.20 (d, 6H).

### Example 35. N-(4-((2-amino-3-chloropyridin-4-yl)oxy)-3-fluorophenyl)-5-ethyl-1-(3-fluoropyridin-2-yl)-1H-pyrazole-4-carboxamide

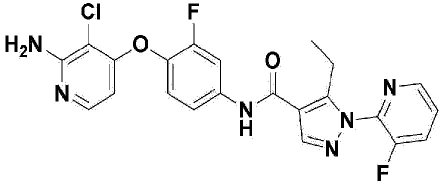

The title compound (10 mg, yield: 18%) was obtained in a manner similar to that of Example 1 using 5-ethyl-1-(3-fluoropyridin-2-yl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.23 (s, 1H), 8.54 (d, 1H), 8.37 (s, 1H), 8.15 (t, 1H), 7.95 (d, 1H), 7.78 (m, 2H), 7.57 (d, 1H), 7.35 (t, 1H), 6.43 (s, 2H), 5.95 (d, 1H), 2.94 (q, 2H), 1.08 (t, 3H); MS *m*/*z:* 471[M+H]

### Example 36. N-(4-((2-amino-3-chloropyridin-4-yl)oxy)-3-fluorophenyl)-1-(3-chloropyridin-2-yl)-5-ethyl-1H-pyrazole-4-carboxamide

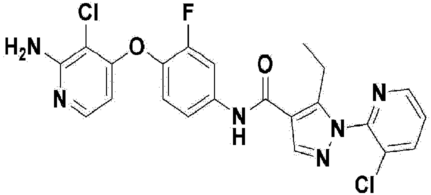

The title compound (15 mg, yield: 26%) was obtained in a manner similar to that of Example 1 using 1-(3-chloropyridin-2-yl)-5-ethyl-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.23 (s, 1H), 8.66 (d, 1H), 8.35 (m, 2H), 7.95 (d, 1H), 7.76 (m, 2H), 7.57 (d, 1H), 7.38 (t, 1H), 6.43 (s, 2H), 5.95 (d, 1H), 2.85 (q, 2H), 1.04 (t, 3H); MS *m*/*z:* 488[M+H]

### Example 37. N-(4-((2-amino-3-iodopyridin-4-yl)oxy)-3-fluorophenyl)-5-ethyl-1-(3-fluoropyridin-2-yl)-1H-pyrazole-4-carboxamide

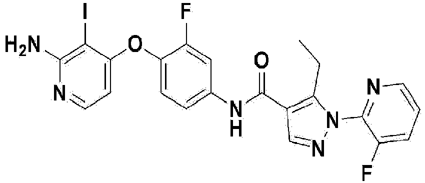

The title compound (230 mg, yield: 71%) was obtained in a manner similar to that of Example 28 using 1-(3-fluoropyridin-2-yl)-5-(trifluoromethyl)-1H-pyrazole-4-carboxylic acid instead of 5-ethyl-1-(3-fluoropyridin-2-yl)-1H-pyrazole-4-carboxylic acid according to Example 28.
¹H NMR (500 MHz, DMSO-d₆) δ 10.22 (s, 1H), 8.53 (d, 1H), 8.38 (s, 1H), 8.15 (t, 1H), 7.94 (d, 1H), 7.78 (m, 2H) 7.57 (d, 1H), 7.32 (t, 1H), 2.95 (q, 2H), 1.08 (t, 3H); MS *m*/*z:* 563[M+H]

### Example 38. N-(4-((2-amino-3-iodopyridin-4-yl)oxy)-3-fluorophenyl)-1-(3-chloropyridin-2-yl)-5-ethyl-1H-pyrazole-4-carboxamide

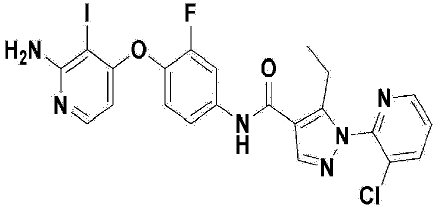

The title compound (230 mg, yield: 71%) was obtained in a manner similar to that of Example 28 using 1-(3-fluoropyridin-2-yl)-5-(trifluoromethyl)-1H-pyrazole-4-carboxylic acid instead of 1-(3-chloropyridin-2-yl)-5-ethyl-1H-pyrazole-4-carboxylic acid according to Example 28.
¹H NMR (500MHz, DMSO-d₆) δ 10.22 (s, 1H), 8.66 (d, 1H), 8.35 (m, 2H), 7.94 (d, 1H), 7.75 (d, 1H), 7.57 (d, 1H), 7.32 (t, 1H), 6.22 (s, 2H), 5.82 (d, 1H), 2.85 (q, 2H), 1.04 (t, 3H); MS *m*/*z:* 579[M+H]

### Example 39. N-(4-((2-amino-3-chloropyridin-4-yl)oxy)-3-fluorophenyl)-1'-methyl-5-(trifluoromethyl)-1'H-[1,4'-bipyrazole]-4-carboxamide

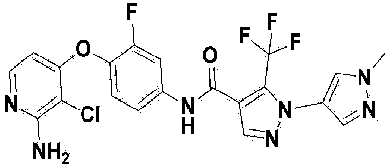

The title compound (34 mg, yield: 44%) was obtained in a manner similar to that of Example 1 using 1'-methyl-5-(trifluoromethyl)-1'H-[1,4'-bipyrazole]-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.87 (s, 1H), 8.28 (m, 2H), 7.90 (d, 1H), 7.78 (m, 2H), 7.53 (m, 1H), 7.42 (m, 1H), 6.94 (brs, 2H), 6.10 (brs, 1H), 3.94 (s, 3H); MS *m*/*z:* 496 [M+H]

### Example 40. N-(4-((2-amino-3-chloropyridin-4-yl)oxy)-3-fluorophenyl)-1-(4-methylthiazol-2-yl)-5-(trifluoromethyl)-1H-pyrazole-4-carboxamide

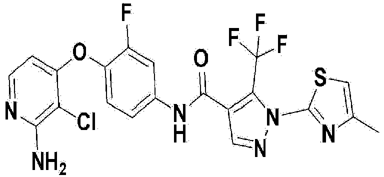

The title compound (8 mg, yield: 10%) was obtained in a manner similar to that of Example 1 using 1-(4-methylthiazol-2-yl)-5-(tirfluoromethyl)-1H-pyrazole-4-carboxylic acid instead of 5-ethyl-1-(pyrimidin-5-yl)-1H-pyrazole-4-carboxylic acid according to Example 1.
¹H NMR (500 MHz, DMSO-d₆) δ 10.97 (s, 1H), 8.42 (s, 1H), 7.87 (d, 1H), 7.76 (m, 1H), 7.50 (m, 2H), 7.39 (m, 1H), 6.42 (brs, 2H), 5.97 (brs, 1H), 2.40 (s, 3H); MS *m*/*z:* 513 [M+H]

### Example 41. N-[4-(6-amino-5-chloro-pyrimidin-4-yl)oxy-3-fluoro-phenyl]-4-phenyl-thiazole-2-carboxamide

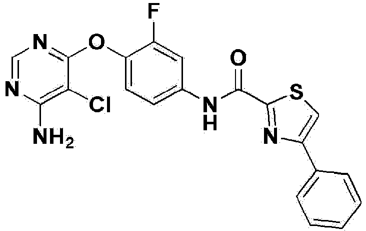

6-(4-amino-2-fluorophenoxy)-5-chloropyrimidine-4-amine (40 mg, 0.16 mmol) was dissolved in 3 ml of THF, and then 1-(4-phenylthiazol-2-yl)-5-(trifluoromethyl)-1H-pyrazole-4-carboxylic acid (53 mg, 0.16 mmol), HATU (90 mg, 0.24 mmol), and DIPEA (41 µl, 0.24 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (4 mg, yield: 4%).
¹H NMR (500 MHz, DMSO-d₆) δ 10.99 (s, 1H), 8.46 (s, 1H), 8.23 (s, 1H), 7.99 (m, 3H), 7.81 (d, 1H), 7.51 (m, 2H), 7.38 (m, 4H); MS *m*/*z:* 576[M+H]

### Example 42. N-[4-(6-amino-5-chloro-pyrimidin-4-yl)oxy-3-fluoro-phenyl]-1-(1-methylpyrazol-4-yl)-5-(trifluoromethyl)pyrazole-4-carboxamide

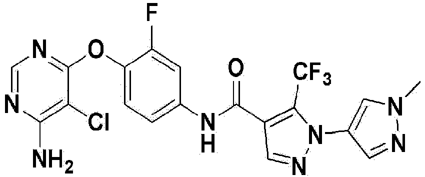

The title compound (58 mg, yield: 75%) was obtained in a manner similar to that of Example 41 using 1'-methyl-5-(trifluoromethyl)-1'H-[1,4'-bipyrazole]-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.79 (s, 1H), 8.28 (m, 2H), 7.96 (s, 1H), 7.80 (m, 2H), 7.47 (m, 1H), 7.42 (m, 3H), 3.94 (s, 3H); MS *m*/*z:* 497[M+H]

### Example 43. N-(4-((6-amino-5-chloropyrimidin-4-yl)oxy)-3-fluorophenyl)-1-(pyridin-2-yl)-5-(trifluoromethyl)-1H-pyrazole-4-carboxamide

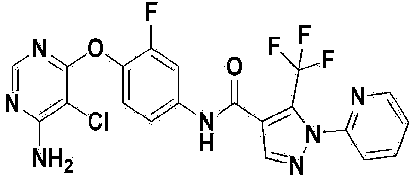

The title compound (48 mg, yield: 62%) was obtained in a manner similar to that of Example 41 using 1-(pyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (400 MHz, DMSO-d₆) δ 10.79 (s, 1H), 8.58 (d, 1H), 8.33 (s, 1H), 8.11 (t, 1H), 7.92 (s, 1H), 7.76 (m, 2H), 7.60 (m, 1H), 7.44 (d, 1H), 7.32 (t, 1H); MS m/z: 494 [M+H]

### Example 44. N-[4-(6-amino-5-chloro-pyrimidin-4-yl)oxy-3-fluoro-phenyl]-1-pyrimidin-5-yl-5-(trifluoromethyl)pyrazole-4-carboxamide

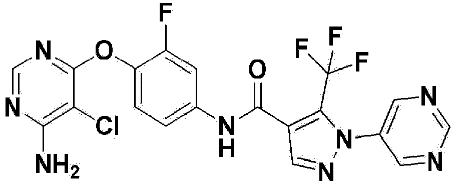

The title compound (40 mg, yield: 52%) was obtained in a manner similar to that of Example 41 using 1-(pyrimidin-5-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 1079 (s, 1H), 9.41 (s, 1H), 9.15 (s, 2H), 8.47 (s, 1H), 7.92 (s, 1H), 7.77 (d, 1H) 7.46 (d, 1H), 7.33 (t, 1H); MS m/z: 495[M+H]

### Example 45. N-[4-(6-amino-5-chloro-pyrimidin-4-yl)oxy-3-fluoro-phenyl]-1-(3-fluoro-2-pyridyl)-5-(trifluoromethyl)pyrazole-4-carboxamide

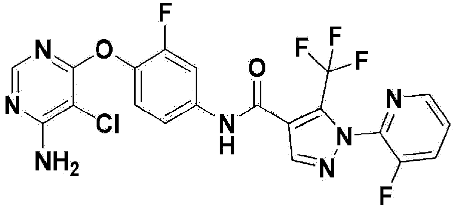

The title compound (39 mg, yield: 49%) was obtained in a manner similar to that of Example 41 using 1-(3-fluoropyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.81 (s, 1H), 8.52 (s, 1H), 8.44 (s, 1H), 8.16 (t, 1H), 7.92 (s, 1H), 7.79 (m, 2H), 7.46 (m, 1H), 7.35 (m, 3H); MS *m*/*z:* 512[M+H]

### Example 46. N-[4-(6-amino-5-chloro-pyrimidin-4-yl)oxy-3-fluoro-phenyl]-1-(3-methyl-2-pyridyl)-5-(trifluoromethyl)pyrazole-4-carboxamide

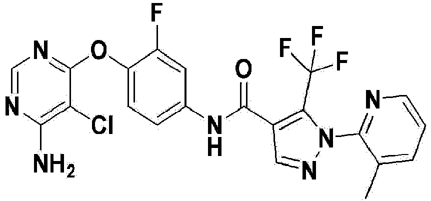

The title compound (31 mg, yield: 39%) was obtained in a manner similar to that of Example 41 using 1-(3-methylpyridin-2-yl)-5-(trifluoromethyl)-1H-pyrazole-4-carboxylic acid obtained in Preparation Example 2 instead of 1-(4-phenylthiazol-2-yl)-5 - (trifluoromethyl)-1H-pyrazole-4-carboxylic acid according to Example 41.
¹H NMR (500 MHz, DMSO-d₆) δ 10.75 (s, 1H), 8.44 (s, 1H), 8.37 (s, 1H), 8.01 (m, 1H), 7.92 (s, 1H), 7.84 (d, 1H), 7.60 (d, 1H), 7.49 (m, 1H), 7.33 (m, 3H), 2.12 (s, 3H); MS *m*/*z:* 508[M+H]

### Example 47. N-[4-(6-amino-5-chloro-pyrimidin-4-yl)oxy-3-fluoro-phenyl]-1-(4-methyl-2-pyridyl)-5-(trifluoromethyl)pyrazole-4-carboxamide

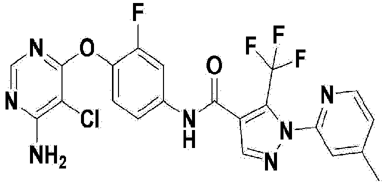

The title compound (45 mg, yield: 56%) was obtained in a manner similar to that of Example 41 using 1-(4-methylpyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.84 (s, 1H), 8.47 (s, 1H), 8.36 (s, 1H), 7.96 (s, 1H), 7.82 (d, 1H), 7.68 (d, 1H), 7.47 (m, 2H), 7.35 (m, 3H), 2.48 (s, 3H); MS *m*/*z:* 508 [M+H]

### Example 48. N-[4-(6-amino-5-chloro-pyrimidin-4-yl)oxy-3-fluoro-phenyl]-1-(5-methyl-2-pyridyl)-5-(trifluoromethyl)pyrazole-4-carboxamide

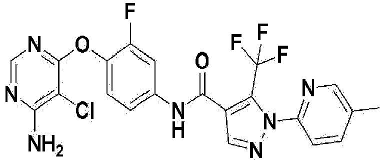

The title compound (52 mg, yield: 65%) was obtained in a manner similar to that of Example 41 using 1-(5-methylpyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.83 (s, 1H), 8.46 (s, 1H), 8.35 (s, 1H), 7.96 (s, 2H), 7.82 (d, 1H), 7.72 (d, 1H), 7.49 (m, 1H), 7.35 (m, 3H), 2.42 (s, 3H); MS *m*/*z:* 508 [M+H]

### Example 49. N-[4-(6-amino-5-chloro-pyrimidin-4-yl)oxy-3-fluoro-phenyl]-1-(6-methyl-2-pyridyl)-5-(trifluoromethyl)pyrazole-4-carboxamide

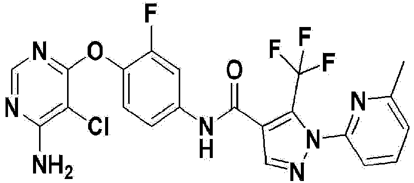

The title compound (50 mg, yield: 63%) was obtained in a manner similar to that of Example 41 using 1-(6-methylpyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.84 (s, 1H), 8.34 (s, 1H), 8.03 (m, 2H), 7.82 (d, 1H), 7.63 (d, 1H), 7.48 (m, 2H), 7.36 (m, 3H), 2.50 (s, 3H); MS *m*/*z:* 508[M+H]

### Example 50. N-[4-(6-amino-5-chloro-pyrimidin-4-yl)oxy-3-fluoro-phenyl]-1-(5-fluoro-2-pyridyl)-5-(trifluoromethyl)pyrazole-4-carboxamide

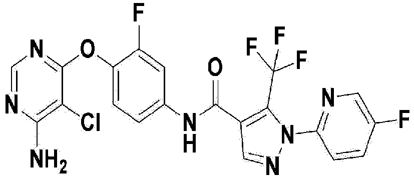

The title compound (39 mg, yield: 49%) was obtained in a manner similar to that of Example 41 using 1-(5-fluoropyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.85 (s, 1H), 8.68 (s, 1H), 8.39 (s, 1H), 8.13 (t, 1H), 7.96 (m, 2H), 7.82 (m, 1H), 7.49 (m, 1H), 7.37 (m, 3H); MS *m*/*z:* 512[M+H]

### Example 51. N-[4-(6-amino-5-chloro-pyrimidin-4-yl)oxy-3-fluoro-phenyl]-1-(3-chloro-2-pyridyl)-5-(trifluoromethyl)pyrazole-4-carboxamide

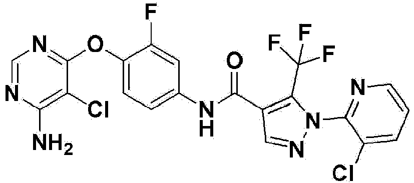

The title compound (42 mg, yield: 51%) was obtained in a manner similar to that of Example 41 using 1-(3-chloropyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.83 (s, 1H), 8.68 (s, 1H), 8.50 (s, 1H), 8.41 (m, 1H), 7.97 (s, 1H), 7.84 (m, 2H), 7.50 (m, 1H), 7.36 (m, 3H); MS *m*/*z:* 529[M+H]

### Example 52. N-[4-(6-amino-5-chloro-pyrimidin-4-yl)oxy-3-fluoro-phenyl]-1-(4-chloro-2-pyridyl)-5-(trifluoromethyl)pyrazole-4-carboxamide

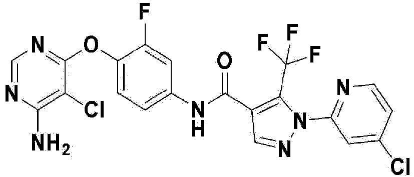

The title compound (56 mg, yield: 67%) was obtained in a manner similar to that of Example 41 using 1-(4-chloropyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.88 (s, 1H), 8.63 (s, 1H), 8.42 (s, 1H), 8.05 (s, 1H), 7.96 (s, 1H), 7.80 (m, 2H), 7.47 (m, 1H), 7.37 (m, 3H); MS *m*/*z:* 529[M+H]

### Example 53. N-[4-(6-amino-5-chloro-pyrimidin-4-yl)oxy-3-fluoro-phenyl]-5-(trifluoromethyl)-1-[3-(trifluoromethyl)-2-pyridyl]pyrazole-4-carboxamide

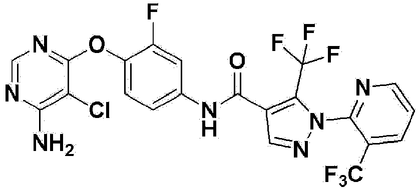

The title compound (65 mg, yield: 74%) was obtained in a manner similar to that of Example 41 using 5-(trifluoromethyl)-1-(3-(trifluoromethyl)pyridin-2-yl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.83 (s, 1H), 8.99 (s, 1H), 8.66 (m, 1H), 8.50 (s, 1H), 8.03 (m, 1H), 7.97 (s, 1H), 7.84 (m, 1H), 7.50 (m, 1H), 7.36 (m, 3H); MS *m*/*z:* 562[M+H]

### Example 54. N-[4-(6-amino-5-chloro-pyrimidin-4-yl)oxy-3-fluoro-phenyl]-1-(3-methoxy-2-pyridyl)-5-(trifluoromethyl)pyrazole-4-carboxamide

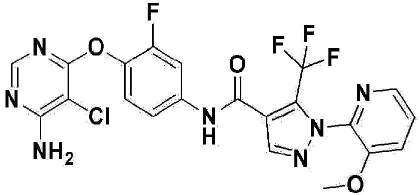

The title compound (56 mg, yield: 68%) was obtained in a manner similar to that of Example 41 using 1-(3-methoxypyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.75 (s, 1H), 8.38 (s, 1H), 8.21 (s, 1H), 7.97 (s, 1H), 7.87 (m, 2H), 7.73 (m, 1H), 7.50 (m, 1H), 7.35 (m, 3H), 3.86 (s, 3H); MS *m*/*z:* 524[M+H]

### Example 55. N-[4-(6-amino-5-chloro-pyrimidin-4-yl)oxy-3-fluoro-phenyl]-1-(4-ethyl-2-pyridyl)-5-(trifluoromethyl)pyrazole-4-carboxamide

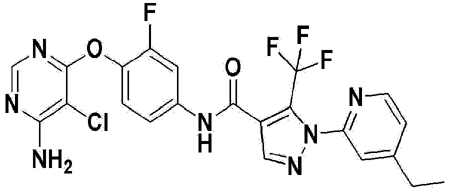

The title compound (42 mg, yield: 51%) was obtained in a manner similar to that of Example 41 using 1-(4-ethylpyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.84 (s, 1H), 8.50 (d, 1H), 8.36 (s, 1H), 7.96 (s, 1H), 7.83 (m, 1H), 7.70 (s, 1H), 7.47 (m, 2H), 7.35 (m, 3H), 2.79 (q, 3H), 1.26 (t, 3H); MS *m*/*z:* 522[M+H]

### Example 56. N-[4-(6-amino-5-chloro-pyrimidin-4-yl)oxy-3-fluoro-phenyl]-1-(3-pyridyl)-5-(trifluoromethyl)pyrazole-4-carboxamide

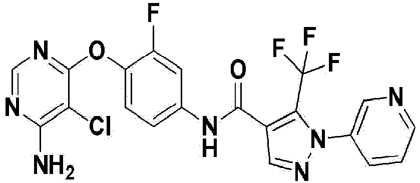

The title compound (43 mg, yield: 55%) was obtained in a manner similar to that of Example 41 using 1-(pyridin-3-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.82 (s, 1H), 8.83 (d, 2H), 8.43 (s, 1H), 8.11 (d, 1H), 7.96 (s, 1H), 7.83 (m, 1H), 7.72 (m, 1H), 7.48 (m, 1H), 7.35 (m, 3H); MS *m*/*z:* 494 [M+H]

### Example 57. N-[4-(6-amino-5-chloro-pyrimidin-4-yl)oxy-3-fluoro-phenyl]-1-(4-pyridyl)-5-(trifluoromethyl)pyrazole-4-carboxamide

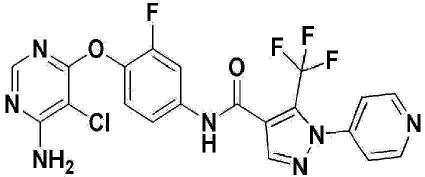

The title compound (40 mg, yield: 52%) was obtained in a manner similar to that of Example 41 using 1-(pyridin-4-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.84 (s, 1H), 8.87 (d, 2H), 8.45 (s, 1H), 7.96 (s, 1H), 7.82 (m, 1H), 7.68 (d, 2H), 7.48 (m, 1H), 7.36 (m, 3H); MS *m*/*z:* 494[M+H]

### Example 58. N-[4-(6-amino-5-chloro-pyrimidin-4-yl)oxy-3-fluoro-phenyl]-1-(3-ethyl-2-pyridyl)-5-(trifluoromethyl)pyrazole-4-carboxamide

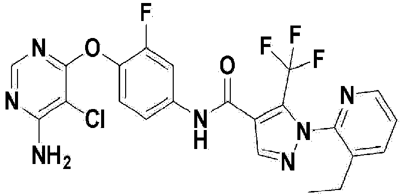

The title compound (42 mg, yield: 51%) was obtained in a manner similar to that of Example 41 using 1-(3-ethylpyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.79 (s, 1H), 8.50 (d, 1H), 8.42 (s, 1H), 8.08 (d, 1H), 7.97 (s, 1H), 7.82 (m, 1H), 7.68 (m, 1H), 7.50 (1, 1H), 7.35 (m, 3H), 2.51 (q, 3H), 1.10 (t, 3H); MS *m*/*z:* 522[M+H]

### Example 59. N-[4-(6-amino-5-chloro-pyrimidin-4-yl)oxy-3-fluoro-phenyl]-1-pyrimidin-2-yl-5-(trifluoromethyl)pyrazole-4-carboxamide

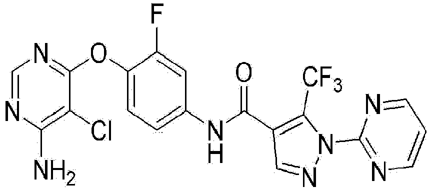

The title compound (21 mg, yield: 54%) was obtained in a manner similar to that of Example 41 using 1-(pyrimidin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.83 (s, 1H), 9.05 (d, 2H), 8.37 (s, 1H), 7.92 (s, 1H), 7.78 (t, 2H), 7.45 (m, 1H), 7.33 (m, 3H); MS *m*/*z:* 495[M+H]

### Example 60. N-[4-(6-amino-5-chloro-pyrimidin-4-yl)oxy-3-fluoro-phenyl]-1-pyridazin-3-yl-5-(trifluoromethyl)pyrazole-4-carboxamide

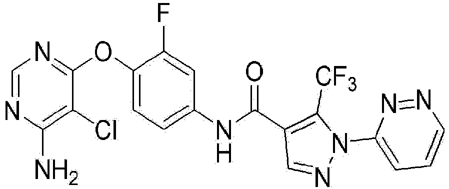

The title compound (57 mg, yield: 73%) was obtained in a manner similar to that of Example 41 using 1-(pyridazin-3-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 1.
¹H NMR (500 MHz, DMSO-d₆) δ 10.86 (s, 1H), 9.43 (d, 1H), 8.47 (s, 1H), 8.19 (d, 1H), 8.08 (d, 1H), 7.92 (s, 1H), 7.73 (m, 1H), 7.47 (m, 1H), 7.33 (m, 3H); MS *m*/*z:* 495[M+H]

### Example 61. N-[4-(6-amino-5-chloro-pyrimidin-4-yl)oxy-3-fluoro-phenyl]-1-pyrimidin-4-yl-5-(trifluoromethyl)pyrazole-4-carboxamide

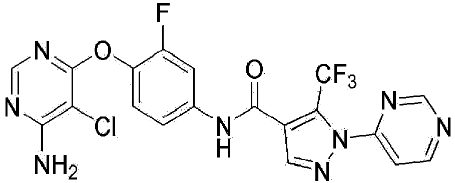

The title compound (37 mg, yield: 48%) was obtained in a manner similar to that of Example 41 using 1-(pyrimidin-4-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.95 (s, 1H), 9.29 (d, 1H), 9.12 (t, 1H), 8.49 (s, 1H), 8.07 (d, 1H), 7.96 (s, 1H), 7.80 (m, 1H), 7.45 (m, 1H), 7.37 (m, 3H); MS *m*/*z:* 495[M+H]

### Example 62. N-[4-(6-amino-5-chloro-pyrimidin-4-yl)oxy-3-fluoro-phenyl]-1-(4-methylpyrimidin-2-yl)-5-(trifluoromethyl)pyrazole-4-carboxamide

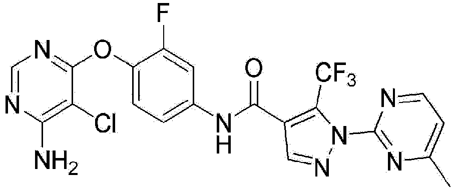

The title compound (25 mg, yield: 62%) was obtained in a manner similar to that of Example 41 using 1-(4-methylpyrimidin-2-yl)-5-(tirfluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.85 (s, 1H), 8.91 (d, 1H), 8.39 (s, 1H), 7.97 (s, 1H), 7.83 (m, 1H), 7.68 (d, 1H), 7.48 (m, 1H), 7.35 (m, 3H), 2.61 (s, 3H); MS *m*/*z:* 509[M+H]

### Example 63. N-[4-(6-amino-5-chloro-pyrimidin-4-yl)oxy-3-fluoro-phenyl]-1-(4-fluoro-2-pyridyl)-5-(trifluoromethyl)pyrazole-4-carboxamide

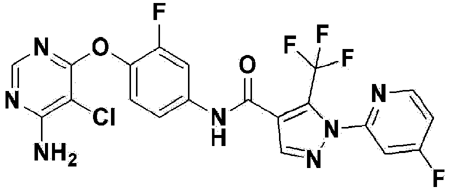

The title compound (45 mg, yield: 56%) was obtained in a manner similar to that of Example 41 using 1-(4-fluoropyridin-2-yl)-5-(trifluoromethyl)-1H-pyrazole-4-carboxylic acid instead of 1-(4-phenylthiazol-2-yl)-5-(trifluoromethyl)-1H-pyrazole-4-carboxylic acid according to Example 41.
¹H NMR (500 MHz, DMSO-d₆) δ 10.87 (s, 1H), 8.69 (d, 1H), 8.41 (s, 1H), 7.96 (s, 1H), 7.81 (m, 2H), 7.61 (t, 1H), 7.47 (m, 1H), 7.35 (m, 3H); MS *m*/*z:* 512[M+H]

### Example 64. N-[4-(6-amino-5-chloro-pyrimidin-4-yl)oxy-3-fluoro-phenyl]-1-(4-hydroxy-2-pyridyl)-5-(trifluoromethyl)pyrazole-4-carboxamide

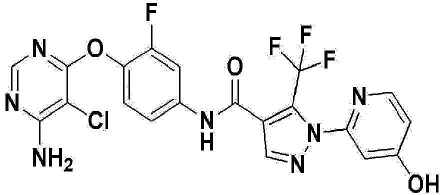

The title compound (37 mg, yield: 46%) was obtained in a manner similar to that of Example 41 using 1-(4-hydroxypyridin-2-yl)-5-(tirfluoromethyl)-1H-pyrazole-4-carboxylic acid instead of 1-(4-phenylthiazol-2-yl)-5-(tirfluoromethyl)-1H-pyrazole-4-carboxylic acid according to Example 41.
¹H NMR (500 MHz, DMSO-d₆) δ 11.42 (s, 1H), 10.80 (s, 1H), 8.31 (m, 2H), 7.96 (s, 1H), 7.82 (m, 1H), 7.48 (m, 1H), 7.34 (m, 3H), 7.13 (s, 1H), 6.97 (d, 1H); MS *m*/*z:* 510[M+H]

### Example 65. N-[4-[5-chloro-6-(cyclopropylamino)pyrimidin-4-yl]oxy-3-fluoro-phenyl]-1-(3-fluoro-2-pyridyl)-5-(trifluoromethyl)pyrazole-4-carboxamide

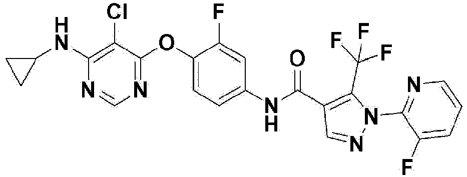

6-(4-amino-2-fluorophenoxy)-5-chloro-N-cyclopropylpyrimidine-4-amine (40 mg, 0.16 mmol) was dissolved in 3 ml of THF, and then 1-(3-fluoropyridin-2-yl)-5-(trifluoromethyl)-1H-pyrazole-4-carboxylic acid (45 mg, 0.16 mmol), HATU (78 mg, 0.20 mmol), and DIPEA (47 µl, 0.27 mmol) were added thereto, followed by stirring at room temperature for 12 hours. The reaction solution was diluted with water, and then extracted three times with ethyl acetate and concentrated. The residue was purified by prep-HPLC (0.1% formic acid in water/acetonitrile) to obtain the title compound (50 mg, yield: 69%).

¹H NMR (400 MHz, DMSO-d₆) δ 10.81 (s, 1H), 8.51 (m, 1H), 8.44 (s, 1H), 8.18 (m, 1H), 8.07 (s, 1H), 7.82 (m, 2H), 7.54 (m, 1H), 7.44 (m, 1H), 7.31 (t, 1H), 2.82 (m, 1H), 0.68 (m,4H).

### Example 66. N-[4-[5-chloro-6-(cyclopropylamino)pyrimidin-4-yl]oxy-3-fluoro-phenyl]-1-(3-chloro-2-pyridyl)-5-(trifluoromethyl)pyrazole-4-carboxamide

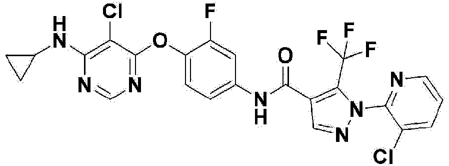

The title compound (70 mg, yield: 91%) was obtained in a manner similar to that of Example 65 using 1-(3-chloropyridin-2-yl)-5-(tirfluoromethyl)-1H-pyrazole-4-carboxylic acid instead of 1-(3-fluoropyridin-2-yl)-5-(trifluoromethyl)-1H-pyrazole-4-carboxylic acid according to Example 65.
¹H NMR (400 MHz, DMSO-d₆) δ 10.78 (s, 1H), 8.63 (m, 1H), 8.44 (s, 1H), 8.36 (m, 1H), 8.07 (s, 1H), 7.76 (m, 2H), 7.54 (m, 1H), 7.44 (m, 1H), 7.30 (t, 1H), 2.82 (m, 1H), 0.68 (m,4H)

### Experimental Example 1. Evaluation of inhibitory activity against RON and MET

### [1-1] Evaluation of inhibitory activity against RON

In order to measure the inhibitory activity of each of the compounds of Examples against RON, the IC50 of each of the compounds was measured using Time-resolved fluorescence energy transfer (TR-FRET).

Specifically, the compound was prepared to a concentration of 1 mM in 100% DMSO and was diluted 10 times in a stepwise manner through 3-fold dilution. The compound (2 ul) diluted in a stepwise manner was added to a 96-well-plate containing 48 ul of a 1x kinase reaction buffer (50 mM HEPES (pH 7.4), 0.01% Tween-20, 5 mM DTT, 0.5 mM Na₃VO₄, 2 mM EGTA, 10 mM MgCl₂).

The RON protein was diluted to a concentration of 49.3 nM in a RON storage buffer (50 mM Tris-HCl (pH 7.5), 150 mM NaCl, 0.5 mM EDTA, 0.02% Triton X-100), and then diluted again to a concentration of 0.4 nM in a 1x kinase reaction buffer. As a substrate cocktail, a RON-specific substrate mixed solution (40uM ULight-labeled Poly GT, 100 nM ATP) was prepared at a concentration 2 times the final reaction concentration.

Thereafter, a 384-well-plate was prepared, and 2.5 µl of each of the diluted compounds of Examples was added to a corresponding well of experimental group wells, and 2.5 µl of a 4% DMSO solution was dispensed into high control wells and low control wells.

Thereafter, 2.5 µL of 0.4 nM RON was dispensed into each of the high control wells and the experimental group wells, and 2.5 µL of the 1x kinase reaction buffer was added to the low control wells, and all the wells were centrifuged at room temperature for 40 seconds at 1,000 RPM, and then incubated at room temperature for 20-30 minutes. Then, 5uL of the substrate cocktail (2x) was dispensed into each well, and all the wells were centrifuged at room temperature for 40 seconds at 1,000 RPM, and then incubated at room temperature for 60 minutes. Thereafter, 5 uL of 30 mM EDTA was added to each well to terminate the reaction, and all the wells were then incubated again at room temperature for 5 minutes. Thereafter, 5 uL of a 4x phosphotyrosine antibody (Perkin Elmer) containing europium was added to each well, and all the wells were then incubated at room temperature for 60 minutes. After the incubation, the 384-well-plate was read with the Vison plate reader. In this case, the excitation wavelength was 340 nm, and the emission wavelength was 620 nm and 665 nm. The IC50 value of each compound of Examples was derived using the GraphPd Prism7 program.

### [1-2] Evaluation of inhibitory activity against MET

In addition, in order to measure the enzyme activity inhibition for cMET, the evaluation was performed in the same manner as the enzyme activity inhibition measurement experiment for RON.

Specifically, the compound was prepared to a concentration of 1 mM in 100% DMSO and was diluted 10 times in a stepwise manner through 3-fold dilution. The compound (2 ul) diluted in a stepwise manner was added to a 96-well-plate containing 48 ul of a 1x kinase reaction buffer (50 mM HEPES (pH 7.4), 0.05% BSA, 0.005 % Tween-20, 1 mM DTT, 0.5 mM MnCl₂, 20 mM MgCl₂).

The cMET protein was diluted to a concentration of 263 nM in a cMET storage buffer (50 mMTris-HCl (pH 7.5), 150 mMNaCl, 0.05% Brij35, 1 mMDTT, 10% Glycerol), and then diluted again to a concentration of 2 nM in a 1x kinase reaction buffer. As a substrate cocktail, a MET-specific substrate mixed solution (5 uM TK peptide, 10 mM ATP) was prepared at a concentration 2 times the final reaction concentration.

Thereafter, a 384-well-plate was prepared, and 2.5 µl of each of the diluted compounds of Examples was added to a corresponding well of experimental group wells, and 2.5 µl of a 4% DMSO solution was dispensed into high control wells and low control wells.

Thereafter, 2.5 µL of 2 nM cMET was dispensed into each of the high control wells and the experimental group wells, and 2.5 µL of the 1x kinase reaction buffer was added to the low control wells, and all the wells were centrifuged at room temperature for 40 seconds at 1,000 RPM, and then incubated at room temperature for 20-30 minutes. Then, 5uL of the substrate cocktail (2x) was dispensed into each well, and all the wells were centrifuged at room temperature for 40 seconds at 1,000 RPM, and then incubated at room temperature for 60 minutes. Thereafter, 5 uL of 90 mM EDTA was added to each well to terminate the reaction, and all the wells were then incubated again at room temperature for 5 minutes. Thereafter, 5 uL of a 4x phosphotyrosine antibody (Perkin Elmer) containing europium was added to each well, and all the wells were then incubated at room temperature for 60 minutes. After the incubation, the 384-well-plate was read with the Vison plate reader. In this case, the excitation wavelength was 340 nm, and the emission wavelength was 620 nm and 665 nm. The IC50 value of each compound of Examples was derived using the GraphPd Prism7 program.

The results of evaluating the inhibitory activity of the compounds of Examples for RON or cMET are shown in Table 2 below.

### (A: < 50 nM, B: 50-500 nM, C: 500-5,000 nM, D: > 5,000 nM)

**[Table 2]**

| | RON (IC₅₀, nM) | MET (IC₅₀, nM) |
|---|---|---|
| Example 1 | B | C |
| Example 2 | A | C |
| Example 3 | D | D |
| Example 4 | C | D |
| Example 5 | B | C |
| Example 6 | B | D |
| Example 7 | B | D |
| Example 8 | A | C |
| Example 9 | A | C |
| Example 10 | A | C |
| Example 11 | B | C |
| Example 12 | B | C |
| Example 13 | B | D |
| Example 14 | B | D |
| Example 15 | B | C |
| Example 16 | B | D |
| Example 17 | B | C |
| Example 18 | B | C |
| Example 19 | B | C |
| Example 20 | A | C |
| Example 21 | A | D |
| Example 22 | B | D |
| Example 23 | B | D |
| Example 24 | B | C |
| Example 25 | A | D |
| Example 26 | B | D |
| Example 27 | B | D |
| Example 28 | A | C |
| Example 29 | A | D |
| Example 30 | C | D |
| Example 31 | A | D |
| Example 32 | A | C |
| Example 33 | B | D |
| Example 34 | A | D |
| Example 35 | A | D |
| Example 36 | B | C |
| Example 37 | A | C |
| Example 38 | A | C |
| Example 39 | B | D |
| Example 40 | B | D |
| Example 41 | D | D |
| Example 42 | C | D |
| Example 43 | A | D |
| Example 44 | B | D |
| Example 45 | A | C |
| Example 46 | B | D |
| Example 47 | B | D |
| Example 48 | C | D |
| Example 49 | C | D |
| Example 50 | B | D |
| Example 51 | A | C |
| Example 52 | C | D |
| Example 53 | B | C |
| Example 54 | B | D |
| Example 55 | C | D |
| Example 56 | B | D |
| Example 57 | C | D |
| Example 58 | B | D |
| Example 59 | A | D |
| Example 60 | B | D |
| Example 61 | C | D |
| Example 62 | B | D |
| Example 63 | B | D |
| Example 64 | B | D |
| Example 65 | C | D |
| Example 66 | D | D |
| A: < 50 nM, B: 50-500 nM, C: 500-5,000 nM, D: > 5,000 nM | | |

## Claims

1. A pyridine derivative compound of Formula 1 below or a pharmaceutically acceptable salt thereof: 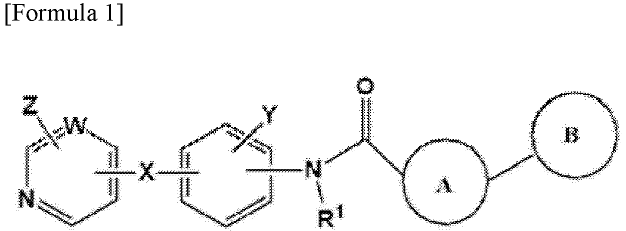 wherein, in the Formula 1 above,
W above is N or CH,
X above is O,
Y above is selected from among hydrogen, halogen, C₁-C₆ alkyl, and C₃-C₆ cycloalkyl,
Z above is at least one selected from among hydrogen, halogen, C₁-C₆ alkyl, C₃-C₁₀ cycloalkyl, C₆-C₁₀ aryl, halogen-substituted C₁-C₆ alkyl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol, and NR²R³,
R¹ above is hydrogen or C₁-C₆ alkyl,
A above represents substituted or unsubstituted 5-membered heteroaryl containing one to four heterocyclic atoms selected from the group consisting of nitrogen, sulfur, and oxygen, wherein the substituent of the substituted 5-membered heteroaryl is halogen, amine, C₁-C₆ alkyl, halogen-substituted C₁-C₆ alkyl, C₆-C₁₀ aryl, C₁-C₆ alkoxy, C₁-C₆ alkylthio, hydroxy, carboxylic acid, C₁-C₆ alkylcarbonyl, C₁-C₆ alkyloxycarbonyl, C₁-C₆ alkylcarbonyloxy, nitro, cyano, carbamoyl, urea, thiol or NR²R³,
B above is C₃-C₁₀ heteroaryl or substituted C₃-C₁₀ heteroaryl, wherein the substituent is halogen, C₁-C₆ alkyl, halogen-substituted C₁-C₆ alkyl, C₁-C₆ alkoxy, hydroxy, or C₆-C₁₀ aryl, and
R² and R³ above are each independently hydrogen, C₁-C₆ alkyl, C₃-C₆ cycloalkyl, substituted C₃-C₆ cycloalkyl,
wherein the halogens are each independently selected from the group consisting of F, Cl, Br, and I.

2. The compound of claim 1 or a pharmaceutically acceptable salt thereof,
wherein the compound is represented by Formula 2 below: 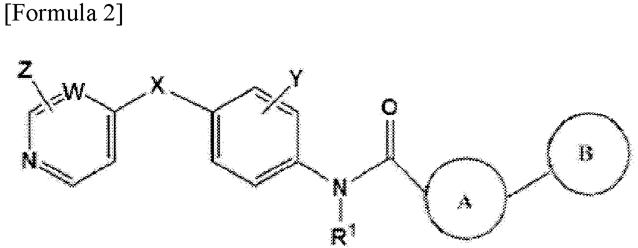
wherein W above is N or CH, Z is at least one selected from hydrogen, halogen, C₁-C₆ alkyl, and NR²R³, wherein R² and R³ above are each independently hydrogen, C₁-C₆ alkyl, C₃-C₆ cycloalkyl, substituted C₃-C₆ cycloalkyl, and the remaining substituents are the same as defined in the Formula 1.

3. The compound of claim 1 or a pharmaceutically acceptable salt thereof, wherein A above includes a heteroaryl group selected from the group consisting of pyrazole, imidazole, thiazole, and thiophene, wherein the heteroaryl group is unsubstituted or substituted by a substituent, wherein the substituent of the substituted heteroaryl group is halogen, amine, C₁-C₆ alkyl, halogen-substituted C₁-C₆ alkyl, or C₆-C₁₀ aryl.

4. The compound of claim 1 or a pharmaceutically acceptable salt thereof, wherein B above is pyridine, C₁-C₃ alkyl-substituted pyridine, halogen-substituted pyridine, halogenated C₁-C₃ alkyl-substituted pyridine, C₁-C₃ alkoxy-substituted pyridine, hydroxy pyridine, pyrimidine, C₁-C₃ alkyl-substituted pyrimidine, halogen-substituted pyrimidine, hydroxy pyrimidine, pyridazine, alkyl-substituted pyridazine, pyrazole, thiazole, alkyl-substituted thiazole, or C₆-C₁₀ aryl-substituted thiazole.

5. The compound of claim 1 or a pharmaceutically acceptable salt thereof, wherein Y above is fluoro.

6. The compound of claim 1 or a pharmaceutically acceptable salt thereof, wherein R¹ above is hydrogen.

7. The compound of claim 1 or a pharmaceutically acceptable salt thereof, wherein th e compound is selected from the group consisting of
N-(4-((2-amino-3-chloropyridin-4-yl)oxy)-3-fluorophenyl)-5-ethyl-1-(pyrimidin-5-yl)-1H-pyrazole-4-carboxamide,
N-(4-((2-amino-3-chloropyridin-4-yl)oxy)-3-fluorophenyl)-1-(pyridin-2-yl)-5-(trifluoromethyl)-1H-pyrazole-4-carboxamide,
N-(4-((2-amino-3-chloropyridin-4-yl)oxy)-3-fluorophenyl)-1-(4-phenylthiazol-2-yl)-5-(trifluoromethyl)-1H-pyrazole-4-carboxamide,
N-(4-((2-amino-3-chloropyridin-4-yl)oxy)-3-fluorophenyl)-5-ethyl-1-(pyridin-4-yl)-1H-pyrazole-4-carboxamide,
N-(4-((2-amino-3-chloropyridin-4-yl)oxy)-3-fluorophenyl)-5-ethyl-1-(pyridin-3-yl)-1H-pyrazole-4-carboxamide,
N-(4-((2-amino-3-chloropyridin-4-yl)oxy)-3-fluorophenyl)-1-(pyrimidin-5-yl)-5-(trifluoromethyl)-1H-pyrazole-4-carboxamide,
N-(4-((2-amino-3-chloropyridin-4-yl)oxy)-3-fluorophenyl)-5-methyl-1-(pyrimidin-5-yl)-1H-pyrazole-4-carboxamide,
N-(4-((2-amino-3-chloropyridin-4-yl)oxy)-3-fluorophenyl)-1-(3-fluoropyridin-2-yl)-5-(trifluoromethyl)-1H-pyrazole-4-carboxamide,
N-(4-((2-amino-3-chloropyridin-4-yl)oxy)-3-fluorophenyl)-1-(3-methylpyridin-2-yl)-5-(trifluoromethyl)-1H-pyrazole-4-carboxamide,
N-(4-((2-amino-3-chloropyridin-4-yl)oxy)-3-fluorophenyl)-1-(3-chloropyridin-2-yl)-5-(trifluoromethyl)-1H-pyrazole-4-carboxamide,
N-(4-((2-amino-3-chloropyridin-4-yl)oxy)-3-fluorophenyl)-5-(trifluoromethyl)-1-(3-(trifluoromethyl)pyridin-2-yl)-1H-pyrazole-4-carboxamide,
N-(4-((2-amino-3-chloropyridin-4-yl)oxy)-3-fluorophenyl)-1-(4-methylpyridin-2-yl)-5-(trifluoromethyl)-1H-pyrazole-4-carboxamide,
N-(4-((2-amino-3-chloropyridin-4-yl)oxy)-3-fluorophenyl)-1-(5-fluoropyridin-2-yl)-5-(trifluoromethyl)-1H-pyrazole-4-carboxamide,
N-(4-((2-amino-3-chloropyridin-4-yl)oxy)-3-fluorophenyl)-1-(4-chloropyridin-2-yl)-5-(trifluoromethyl)-1H-pyrazole-4-carboxamide,
N-(4-((2-amino-3-chloropyridin-4-yl)oxy)-3-fluorophenyl)-1-(3-methoxypyridin-2-yl)-5-(trifluoromethyl)-1H-pyrazole-4-carboxamide,
N-(4-((2-amino-3-chloropyridin-4-yl)oxy)-3-fluorophenyl)-1-(5-methylpyridin-2-yl)-5-(trifluoromethyl)-1H-pyrazole-4-carboxamide,
N-(4-((2-amino-3-chloropyridin-4-yl)oxy)-3-fluorophenyl)-1-(6-methylpyridin-2-yl)-5-(trifluoromethyl)-1H-pyrazole-4-carboxamide,
N-(4-((2-amino-3-chloropyridin-4-yl)oxy)-3-fluorophenyl)-1-(pyridin-3-yl)-5-(trifluoromethyl)-1H-pyrazole-4-carboxamide,
N-(4-((2-amino-3-chloropyridin-4-yl)oxy)-3-fluorophenyl)-1-(pyridin-4-yl)-5-(trifluoromethyl)-1H-pyrazole-4-carboxamide,
N-(4-((2-amino-3-chloropyridin-4-yl)oxy)-3-fluorophenyl)-1-(pyridazin-3-yl)-5-(trifluoromethyl)-1H-pyrazole-4-carboxamide,
N-(4-((2-amino-3-chloropyridin-4-yl)oxy)-3-fluorophenyl)-1-(pyrimidin-2-yl)-5-(trifluoromethyl)-1H-pyrazole-4-carboxamide,
N-(4-((2-amino-3-chloropyridin-4-yl)oxy)-3-fluorophenyl)-1-(pyrimidin-4-yl)-5-(trifluoromethyl)-1H-pyrazole-4-carboxamide,
N-(4-((2-amino-3-chloropyridin-4-yl)oxy)-3-fluorophenyl)-1-(4-methylpyrimidin-2-yl)-5-(trifluoromethyl)-1H-pyrazole-4-carboxamide,
N-(4-((2-amino-3-chloropyridin-4-yl)oxy)-3-fluorophenyl)-1-(3-ethoxypyridin-2-yl)-5-(trifluoromethyl)-1H-pyrazole-4-carboxamide,
N-(4-((2-amino-3-chloropyridin-4-yl)oxy)-3-fluorophenyl)-1-(4-fluoropyridin-2-yl)-5-(trifluoromethyl)-1H-pyrazole-4-carboxamide,
N-(4-((2-amino-3-chloropyridin-4-yl)oxy)-3-fluorophenyl)-1-(5-fluoropyrimidin-2-yl)-5-(trifluoromethyl)-1H-pyrazole-4-carboxamide,
N-(4-((2-amino-3-chloropyridin-4-yl)oxy)-3-fluorophenyl)-1-(5-hydroxypyrimidin-2-yl)-5-(trifluoromethyl)-1H-pyrazole-4-carboxamide,
N-(4-((2-amino-3-iodopyridin-4-yl)oxy)-3-fluorophenyl)-1-(3-fluoropyridin-2-yl)-5-(trifluoromethyl)-1H-pyrazole-4-carboxamide,
N-(4-((2-amino-3-chloropyridin-4-yl)oxy)-3-fluorophenyl)-1-(3,5-difluoropyridin-2-yl)-5-(trifluoromethyl)-1H-pyrazole-4-carboxamide,
N-(4-((2-amino-3-chloropyridin-4-yl)oxy)-3-fluorophenyl)-1-(6-fluoropyridazin-3-yl)-5-(trifluoromethyl)-1H-pyrazole-4-carboxamide,
N-(4-((2-aminopyridin-4-yl)oxy)-3-fluorophenyl)-1-(3-fluoropyridin-2-yl)-5-(trifluoromethyl)-1H-pyrazole-4-carboxamide,
N-(4-((2-amino-3-chloropyridin-4-yl)oxy)-2-fluorophenyl)-1-(3-fluoropyridin-2-yl)-5-(trifluoromethyl)-1H-pyrazole-4-carboxamide,
N-(4-((2-amino-3-(cyclohexylamino)pyridin-4-yl)oxy)-3-fluorophenyl)-1-(3-fluoropyridin-2-yl)-5-(trifluoromethyl)-1H-pyrazole-4-carboxamide,
N-(4-((2-amino-3-(isopropylamino)pyridin-4-yl)oxy)-3-fluorophenyl)-1-(3-fluoropyridin-2-yl)-5-(trifluoromethyl)-1H-pyrazole-4-carboxamide,
N-(4-((2-amino-3-chloropyridin-4-yl)oxy)-3-fluorophenyl)-5-ethyl-1-(3-fluoropyridin-2-yl)-1H-pyrazole-4-carboxamide,
N-(4-((2-amino-3-chloropyridin-4-yl)oxy)-3-fluorophenyl)-1-(3-chloropyridin-2-yl)-5-ethyl-1H-pyrazole-4-carboxamide,
N-(4-((2-amino-3-iodopyridin-4-yl)oxy)-3-fluorophenyl)-5-ethyl-1-(3-fluoropyridin-2-yl)-1H-pyrazole-4-carboxamide,
N-(4-((2-amino-3-iodopyridin-4-yl)oxy)-3-fluorophenyl)-1-(3-chloropyridin-2-yl)-5-ethyl-1H-pyrazole-4-carboxamide,
N-(4-((2-amino-3-chloropyridin-4-yl)oxy)-3-fluorophenyl)-1'-methyl-5-(trifluoromethyl)-1'H-[1,4'-bipyrazole]-4-carboxamide,
N-(4-((2-amino-3-chloropyridin-4-yl)oxy)-3-fluorophenyl)-1-(4-methylthiazol-2-yl)-5-(trifluoromethyl)-1H-pyrazole-4-carboxamide,
N-[4-(6-amino-5-chloro-pyrimidin-4-yl)oxy-3-fluoro-phenyl]-4-phenyl-thiazole-2-carboxamide,
N-[4-(6-amino-5-chloro-pyrimidin-4-yl)oxy-3-fluoro-phenyl]-1-(1-methylpyrazol-4-yl)-5-(trifluoromethyl)pyrazole-4-carboxamide,
N-(4-((6-amino-5-chloropyrimidin-4-yl)oxy)-3-fluorophenyl)-1-(pyridin-2-yl)-5-(trifluoromethyl)-1H-pyrazole-4-carboxamide,
N-[4-(6-amino-5-chloro-pyrimidin-4-yl)oxy-3-fluoro-phenyl]-1-pyrimidin-5-yl-5-(trifluoromethyl)pyrazole-4-carboxamide,
N-[4-(6-amino-5-chloro-pyrimidin-4-yl)oxy-3-fluoro-phenyl]-1-(3-fluoro-2-pyridyl)-5-(trifluoromethyl)pyrazole-4-carboxamide,
N-[4-(6-amino-5-chloro-pyrimidin-4-yl)oxy-3-fluoro-phenyl]-1-(3-methyl-2-pyridyl)-5-(trifluoromethyl)pyrazole-4-carboxamide,
N-[4-(6-amino-5-chloro-pyrimidin-4-yl)oxy-3-fluoro-phenyl]-1-(4-methyl-2-pyridyl)-5-(trifluoromethyl)pyrazole-4-carboxamide,
N-[4-(6-amino-5-chloro-pyrimidin-4-yl)oxy-3-fluoro-phenyl]-1-(5-methyl-2-pyridyl)-5-(trifluoromethyl)pyrazole-4-carboxamide,
N-[4-(6-amino-5-chloro-pyrimidin-4-yl)oxy-3-fluoro-phenyl]-1-(6-methyl-2-pyridyl)-5-(trifluoromethyl)pyrazole-4-carboxamide,
N-[4-(6-amino-5-chloro-pyrimidin-4-yl)oxy-3-fluoro-phenyl]-1-(5-fluoro-2-pyridyl)-5-(trifluoromethyl)pyrazole-4-carboxamide,
N-[4-(6-amino-5-chloro-pyrimidin-4-yl)oxy-3-fluoro-phenyl]-1-(3-chloro-2-pyridyl)-5-(trifluoromethyl)pyrazole-4-carboxamide,
N-[4-(6-amino-5-chloro-pyrimidin-4-yl)oxy-3-fluoro-phenyl]-1-(4-chloro-2-pyridyl)-5-(trifluoromethyl)pyrazole-4-carboxamide,
N-[4-(6-amino-5-chloro-pyrimidin-4-yl)oxy-3-fluoro-phenyl]-5-(trifluoromethyl)-1-[3-(trifluoromethyl)-2-pyridyl]pyrazole-4-carboxamide,
N-[4-(6-amino-5-chloro-pyrimidin-4-yl)oxy-3-fluoro-phenyl]-1-(3-methoxy-2-pyridyl)-5-(trifluoromethyl)pyrazole-4-carboxamide,
N-[4-(6-amino-5-chloro-pyrimidin-4-yl)oxy-3-fluoro-phenyl]-1-(4-ethyl-2-pyridyl)-5-(trifluoromethyl)pyrazole-4-carboxamide,
N-[4-(6-amino-5-chloro-pyrimidin-4-yl)oxy-3-fluoro-phenyl]-1-(3-pyridyl)-5-(trifluoromethyl)pyrazole-4-carboxamide,
N-[4-(6-amino-5-chloro-pyrimidin-4-yl)oxy-3-fluoro-phenyl]-1-(4-pyridyl)-5-(trifluoromethyl)pyrazole-4-carboxamide,
N-[4-(6-amino-5-chloro-pyrimidin-4-yl)oxy-3-fluoro-phenyl]-1-(3-ethyl-2-pyridyl)-5-(trifluoromethyl)pyrazole-4-carboxamide,
N-[4-(6-amino-5-chloro-pyrimidin-4-yl)oxy-3-fluoro-phenyl]-1-pyrimidin-2-yl-5-(trifluoromethyl)pyrazole-4-carboxamide,
N-[4-(6-amino-5-chloro-pyrimidin-4-yl)oxy-3-fluoro-phenyl]-1-pyridazin-3-yl-5-(trifluoromethyl)pyrazole-4-carboxamide,
N-[4-(6-amino-5-chloro-pyrimidin-4-yl)oxy-3-fluoro-phenyl]-1-pyrimidin-4-yl-5-(trifluoromethyl)pyrazole-4-carboxamide,
N-[4-(6-amino-5-chloro-pyrimidin-4-yl)oxy-3-fluoro-phenyl]-1-(4-methylpyrimidin-2-yl)-5-(trifluoromethyl)pyrazole-4-carboxamide,
N-[4-(6-amino-5-chloro-pyrimidin-4-yl)oxy-3-fluoro-phenyl]-1-(4-fluoro-2-pyridyl)-5-(trifluoromethyl)pyrazole-4-carboxamide,
N-[4-(6-amino-5-chloro-pyrimidin-4-yl)oxy-3-fluoro-phenyl]-1-(4-hydroxy-2-pyridyl)-5-(trifluoromethyl)pyrazole-4-carboxamide,
N-[4-[5-chloro-6-(cyclopropylamino)pyrimidin-4-yl]oxy-3-fluoro-phenyl]-1-(3-fluoro-2-pyridyl)-5-(trifluoromethyl)pyrazole-4-carboxamide, and
N-[4-[5-chloro-6-(cyclopropylamino)pyrimidin-4-yl]oxy-3-fluoro-phenyl]-1-(3-chloro-2-pyridyl)-5-(trifluoromethyl)pyrazole-4-carboxamide.

8. A pharmaceutical composition comprising the compound according to any one of claims 1 to 7 or a pharmaceutically acceptable salt thereof; and a pharmaceutically acceptable carrier.
